Europäisches Patentamt

European Patent Office

Office européen des brevets

(19)

(11) **EP 1 282 637 B1**

(12) **EUROPÄISCHE PATENTSCHRIFT**

(45) Veröffentlichungstag und Bekanntmachung des Hinweises auf die Patenterteilung:
**10.03.2004 Patentblatt 2004/11**

(21) Anmeldenummer: **01951459.5**

(22) Anmeldetag: **25.04.2001**

(51) Int Cl.⁷: **C07K 1/34**

(86) Internationale Anmeldenummer:
**PCT/EP2001/004634**

(87) Internationale Veröffentlichungsnummer:
**WO 2001/085757 (15.11.2001 Gazette 2001/46)**

(54) **REINIGUNG VON PROTEINEINSCHLUSSKÖRPERN DURCH QUERSTROM-MIKROFILTRATION**

PURIFICATION OF PROTEIN INCLUSION BODIES BY CROSSFLOW MICROFILTRATION

PURIFICATION DE CORPS D'INCLUSION PROTEIQUE PAR MICRO-FILTRAGE A COURANT TRANSVERSAL

(84) Benannte Vertragsstaaten:
**DE ES FR GB IT**

(30) Priorität: **08.05.2000 DE 10022258**

(43) Veröffentlichungstag der Anmeldung:
**12.02.2003 Patentblatt 2003/07**

(73) Patentinhaber: **Bayer HealthCare AG
51368 Leverkusen (DE)**

(72) Erfinder: **PETERS, Jörg
42781 Haan (DE)**

(56) Entgegenhaltungen:
• **S G WALKER & A LYDDIATT: "Aqueous two-phase systems as an alternative process route for the fractionation of small inclusion bodies" JOURNAL OF CHROMATOGRAPHY B : BIOMEDICAL APPLICATIONS., Bd. 711, 1998, Seiten 185-194, XP002184943 ELSEVIER SCIENCE PUBLISHERS, NL ISSN: 0378-4347**
• **S M BAILEY & M M MEAGHER: "Separation of soluble protein from inclusion bodies in Escherichia coli lysate using crossflow microfiltration" JOURNAL OF MEMBRANE SCIENCE., Bd. 166, 2000, Seiten 137-146, XP002184944 ELSEVIER SCIENTIFIC PUBL.COMPANY. AMSTERDAM., NL ISSN: 0376-7388**
• **M M MEAGHER ET AL.: "Extraction of rIL-2 inclusion bodies from Escherichia coli using cross-flow filtration" BIOTECHNOLOGY AND BIOENGINEERING, Bd. 43, 1994, Seiten 969-977, XP002184945 JOHN WILEY & SONS. NEW YORK., US ISSN: 0006-3592 in der Anmeldung erwähnt**
• **S M FORMAN ET AL.: "Crossflow filtration for the separation of inclusion bodies from soluble proteins in recombinant Escherichia coli cell lysate" JOURNAL OF MEMBRANE SCIENCE., Bd. 48, 1990, Seiten 263-279, XP002184946 ELSEVIER SCIENTIFIC PUBL.COMPANY. AMSTERDAM., NL ISSN: 0376-7388 in der Anmeldung erwähnt**
• **DATABASE CA [Online] CHEMICAL ABSTRACTS SERVICE, COLUMBUS, OHIO, US; GRAHAM, E. R. ET AL: "Fractionation of proteins using ultrafiltration membranes" retrieved from STN Database accession no. 129:78738 CA XP002184947 & ICHEME RES. EVENT, TWO-DAY SYMP. (1998), 1223-1230 PUBLISHER: INSTITUTION OF CHEMICAL ENGINEERS, RUGBY, UK., 1998,**

## Beschreibung

Die Erfindung betrifft ein Verfahren zur Reinigung und/oder zur Konzentrierung von Proteineinschlusskörpern (engl. "Inclusion bodies" oder "refractile bodies") in einer hochkonzentrierten, partikelhaltigen Lösung, wobei man die Proteineinschlusskörper enthaltende suspension tangential an einer oder mehreren semipermeablen Membranen vorbeileitet, so dass die Proteineinschlusskörper hinter den Membranen zurückgehalten werden und Substanzen mit einem kleineren Molekulargewicht durch die Membran durchtreten können und/oder an der Membran adsorbiert werden und man eine gereinigte und/oder konzentrierte Proteineinschlusskörper-suspension erhält. In derselben Weise wird zur Abtrennung von Zellwandpartikeln aus einer Proteineinschlusskörper-Suspension verfahren. Weiter betrifft die Erfindung die Verwendung einer Querstrom-Mikrofiltrationsanlage zur Reinigung und/oder Konzentrierung von Proteineinschlusskörpem in einer Suspension sowie zur Abtrennung von Zellwandpartikeln aus einer Proteineinschlusskörper-Präparation.

### *Beschreibung*

Die vorliegende Erfindung betrifft ein Verfahren zur Reinigung und/oder zur Konzentrierung von Proteineinschlusskörpern in einer hochkonzentrierten, partikelhaltigen Lösung, ein Verfahren zur Abtrennung von Zellwandpartikeln aus einer Proteineinschlusskörper-Suspension sowie die Verwendung einer Querstrom-Mikrofiltrationsanlage zur Reinigung und/oder zur Konzentrierung von Proteineinschlusskörpern in einer Suspension und zur Abtrennung von Zellwandpartikeln aus einer Proteineinschlusskörper-Präparation.

### *Begriffsdefinitionen*

*Proteineinschlusskörper* (engl. "Inclusion bodies" oder "refractile bodies") werden oft bei hoher Expression heterologer oder homologer Protein in *Escherichia coli* gebildet und bestehen aus hochaggregierten Proteinen, Nukleinsäuren, Enzymen der Proteinbiosynthese sowie Ribosomen. Proteineinschlusskörper sind elektronendichte, amorphe Partikel, die gegenüber dem Zytoplasma eine diskrete Grenze aufweisen, aber nicht von einer Membran umhüllt sind (Schoemaker JM et al. (1985): EMBO J 4: 775-780). Während der Präparation der Proteineinschlusskörper können durch verschiedenartige Interaktionen sekundär andere Kontaminationen adsorbiert werden wie z.B. Endotoxine, Zellwandtrümmer und Lipide (Marston FAO (1986): Biochem. J. 240: 1-12).

*Biomasse* bezeichnet hierin die Summe aller in einem Zellaufschluss vorkommenden hochmolekularen wie niedermolekularen Bestandteile. Insbesondere der Anteil an Zellwandtrümmern (Zellwandpartikeln) sowie an Proteineinschlusskörpern, aber auch Bestandteile wie Nukleinsäuren, Endotoxine, lösliches Protein und niedermolekulare Komponenten, bestimmen die physikochemischen Eigenschaften des Filtrationsgutes. Angaben zu Biomassekonzentrationen beziehen sich stets auf die Konzentration an Zellennassgewicht vor dem Zellaufschluss. Man spricht von sog. Biomasse-Äquivalent.

Der *Transmembrandruck (TMP)* ist definiert als:

$$\text{TMP} = (\text{Eingangsdruck} + \text{Ausgangsdruck})/2 - \text{Permeatdruck [bar]}$$

Der *Eingangsdruck* ist der Druck, der am Retentateingang (Eintrittsöffnung der Kassette) anliegt. Der *Ausgangsdruck* ist der Druck, der am Retentatausgang (Austrittsöffnung der Kassette) anliegt. Der *Permeatdruck* ist der Druck, der am Permeat-(Filtrat)-ausgang anliegt.

Unter *Transmission* wird hierin die Abreicherung von löslichen Bestandteilen über die Membran ins Permeat verstanden. Es werden dazu Proben vom Permeat und Retentat zu verschiedenen Zeitpunkten (x) während der Diafiltration gezogen, zentrifugiert (14.000 x g, 15 min) und der lösliche Überstand wird, nach geeigneter Verdünnung, photometrisch bei 280 nm vermessen (optische Dichte bei 280 nm). Die $OD_{280}$-Werte des Permeats zu jedem Zeitpunkt x ($OD_{280}$(Permeat(x)) werden dann zu den $OD_{280}$-Werten des Retentats zu jedem Zeitpunkt x ($OD_{280}$(Retentat(x)) nach folgender Formel in Beziehung gesetzt:

$$\text{Transmission} = OD_{280}(\text{Permeat(x)} / OD_{280}(\text{Retentat(x)} \times 100$$

Die Transmission ändert sich typischerweise im Verlauf einer Diafiltration. Am Beginn ist die Membran noch frei von adsorbierten Bestandteilen und weist keine Deckschicht auf. Daher ist die Transmission definitionsgemäß am Beginn der Filtration 100%. Sie nimmt jedoch während der Filtrationsdauer typischerweise ab, da sich mehr und mehr Deckschicht ausbildet, die den Transport von löslichen Bestandteilen über die Membran hinweg ins Permeat verhindert.

Die Transmission kann also summarisch nur als Mittelwert (mittl. Transmission) angegeben werden.

Unter *Entfernung* wird hierin die Abreicherung von löslichen Bestandteilen aus dem Retentat verstanden. Es werden dabei Proben vom Retentat zu verschiedenen Zeitpunkten (x) während der Diafiltration gezogen, zentrifugiert (14.000 x g, 15 min) und der lösliche Überstand wird, nach geeigneter Verdünnung, photometrisch bei 280 nm vermessen (optische Dichte bei 280 nm). Die $OD_{280}$-Werte ($OD_{280}$(Retentat(x))) werden dann nach folgender Formel in Beziehung gesetzt zu der ursprünglich im Retentat vorhandenen $OD_{280}$ des löslichen Überstands ($OD_{280}$(Retentat(0))):

$$Entfernung = 100 - (OD_{280}(Retentat(x) / OD_{280}(Retentat(0) \times 100)$$

Die Entfernung strebt typischerweise bis zum Ende der Diafiltration auf einen Maximalwert zu. Die Werte stellen also die maximal erreichte Abreicherung löslicher Bestandteile dar.

### Stand der Technik

Im Bereich der Proteinchemie sind Proteineinschlusskörper-Aufreinigungsverfahren gebräuchliche Methoden. In Laborverfahren, die aus dem Stand der Technik bekannten sind, wird z. B. das gewonnene biologische Material (*E. coli* Bakterienzellen), nach dessen Aufschluss (üblicherweise mit enzymatischer Lyse durch Lysozym, Ultraschallbehandlung oder Hochdruckhomogenisation) abzentrifugiert und das gewonnene Sediment, das die Proteineinschlusskörper und Kontaminationen wie unaufgeschlossene Zellen, Zellhüllen und Zellwandfragmente enthält, mit Hilfe der Sedimentation oder, alternativ, durch Querstrom-Mikrofiltration mit Puffer gewaschen. Obschon das Verfahren der Sedimentation generell zur Reinigung von Proteineinschlusskörpem nach deren Freisetzung aus den Zellen genutzt wird (z.B. Schoner RG et al. (1985): Biotechnology 3: 151-154; Sharma SK et al. (1986): J. Biotechnol. 4: 119-124), kann dieses Verfahren im größeren Maßstab Schwierigkeiten bereiten (Forman SM et al. (1990): J. Membr. Sci. 48: 263-279). Die Proteineinschlusskörper können mit Hilfe der Querstrom-Mikrofiltration bis zu einer gewünschten Reinheit gewaschen werden (Meagher MM et al. (1994): Biotechnol. Bioeng. 43: 969-977; Forman SM et al. (1990): J. Membr. Sci. 48: 263-279).

Die mittlere Partikelgröße von Proteineinschlusskörpern ist abhängig vom jeweiligen exprimierten Zielprotein, dem Wirtsstamm, dem Expressionssystem sowie dem verwendeten Kulturmedium und kann im Bereich von 0.07μm für humanes Wachstumshormon (Blum P et al. (1992): Bio/Technology 10: 301-304) bis 1.5 μm für b-Lactamase liegen (Bowden GA et al. (1991): Bio/Technology 9: 725-730). Weitere Beispiele aus der Literatur sind Prochymosin-Proteineinschlusskörper mit Partikeldurchmessern von 1.26 μm (±1.2 μm) sowie Interferon mit 0.81 μm (±0.4 μm) (Taylor G et al. (1986): Bio/Technology 4: 553-557). Zellwandtrümmer hingegen weisen in Abhängigkeit vom Aufschlussverfahren Partikelgrößen zwischen 0.05 μm bis 1 μm auf (Bailey SM and Meagher MM (1997): Biotechnol. Bioeng. 56(3): 304-310). Darüberhinaus hat auch der Aufschlussgrad Einfluss auf die Partikelgrößenverteilung im Rohhomogenat. Somit liegen bei vielen Rohhomogenaten überlappende Partikelgrößenverteilungen zwischen Proteineinschlusskörpern, Zellwandtrümmern und unaufgeschlossenen Zellen vor.

Forman et al. geben eine logarithmische Beziehung zwischen dem Permeatflux und der steigenden Konzentration von Biomasse in der zu filtrierenden Produktlösung an. Das heißt, dass der Permeatflux drastisch abfällt, wenn die initiale Biomassekonzentration in der zu filtrierenden Produktlösung ansteigt. Forman et al. geben als maximale Permeatfluxe 14 L/h m² bei 25 g/L (2.5%) Biomasse-Äquivalent, 12 L/h m² bei 50 g/L (5%) Biomasse-Äquivalent und <8 L/h m² bei 100 g/L (10%) Biomasse-Äquivalent für eine Durapore Membran (Millipore) mit einer Auschlussgrenze von 0.45 μm an. Die Biomassebelastung der Membran lag bei diesen Experimenten bei etwa 1.4 kg Zellfeuchtgewicht-Äquivalent/m². Die Erhöhung des Permeatfluxes über einen bestimmten Grenzwert hinaus resultiert bei gegebener Biomassekonzentration in der Produktlösung in einem dramatischen Anstieg des TMP und damit im Zusetzen der Membranporen (sog. "Fouling" (engl.)). Umgekehrt fällt der Permeatflux bei gegebenem TMP über die Filtrationsdauer ab.

Andere Arbeitsgruppen (Bailey SM & Meagher MM (1997): J. Membr. Sci. 131: 29-38) geben an, dass bereits bei Biomassebelastungen von 4-6% (40-60 g/L) der Permeatflux und die Transmission bei Polysulfon-Hohlfasermodulen oder Polyvinylidenfluorid (PVDF)-Kassettenmodulen über die Zeit abfällt, was durch den Zusatz von chaotropen Agenzien wie Guanidin-Hydrochlorid noch verstärkt wird.

Ähnliche Ergebnisse wurden auch von Meagher und Mitarbeitern (Meagher MM et al. (1994): Biotechnol. Bioeng. 43: 969-977) für die Reinigung von IL-2 Proteineinschlusskörpern auf einer Durapore-Membran (Polyethersulfon, 0.1 μm) berichtet. Bei Biomassekonzentration von 7% (70 g/L) Zellfeuchtgewicht-Äquivalent im Retentat nimmt sowohl die Proteintransmission als auch der Permeatflux (20-10 L/h m²) signifikant über die Filtrationszeit ab, was auf die Bildung und Kompression einer Membrandeckschicht zurückgeführt wurde. Die Adsorption von Protein auf der Membran hängt sowohl von den physikochemischen Eigenschaften der Proteinlösung, als auch von denen der Membran selbst ab.

Aus dem Stand der Technik sind somit keine Verfahren bekannt die es ermöglichen, bei sehr hohen Konzentrationen an Biomasse bei konstanten Betriebsparametern (Druck, Permeatflux, Retentatfluss) Proteineinschlusskörper-Lösungen zu filtrieren. Aus der Literatur sind keine direkten Angaben zur Reproduzierbarkeit der charakteristischen Filtrationsparameter über viele Filtrationszyklen zu entnehmen. Dies hat die Umsetzung von Querstrom-Mikrofiltrationsverfahren zur Feinreinigung von Proteineinschlusskörper-haltigen Suspensionen in den technischen Maßstab bisher verhindert.

***Gegenstand der Erfindung***

Eine der vorliegenden Erfindung zugrundeliegende Aufgabe war es daher, ein Reinigungs- und Konzentrierungsverfahren auf Basis der Querstrom-Mikrofiltration bereitzustellen, das es auf einfache Weise ermöglicht, Proteineinschlusskörper in technisch relevanten Mengen unter konstanten Betriebsparametern während eines und über viele Filtrationszyklen aufzureinigen. Eine weitere der vorliegenden Erfindung zugrundeliegende Aufgabe bestand darin, die Konzentration an Zellwandpartikeln und anderen, niedermolekularen Bestandteilen soweit herabzusenken, dass nachfolgende Aufarbeitungsschritte, insbesondere die Rückfaltung des denaturierten Zielproteins sowie die chromatographische Feinreinigung, mit technisch relevanten Ausbeuten reproduzierbar gelingt.

In einem ersten Aspekt betrifft die vorliegende Erfindung ein Verfahren zur Reinigung und/oder zur Konzentrierung von Proteineinschlusskörpern in einer Lösung, welches dadurch gekennzeichnet ist, dass die Proteineinschlusskörper enthaltende Lösung tangential an einer oder mehreren semipermeablen Membranen vorbeigeleitet wird, so dass die Proteineinschlusskörper von den Membranen zurückgehalten werden und Substanzen oder Bestandteile mit einem kleineren Molekulargewicht bzw. Partikeldurchmesser durchtreten können, wobei eine gereinigte und/oder konzentrierte Proteineinschlusskörper-Lösung erhalten wird.

In einem weiteren Aspekt betrifft die vorliegende Erfindung ein Verfahren zur Abtrennung von Zellwandpartikeln aus einer Proteineinschlusskörper-Präparation, welches dadurch gekennzeichnet ist, dass die Proteineinschlusskörper enthaltende Präparation tangential an einer oder mehreren semipermeablen Membranen vorbeigeleitet wird, so dass die Proteineinschlusskörper von den Membranen zurückgehalten werden und Substanzen oder Bestandteile mit einem kleineren Molekulargewicht bzw. Partikeldurchmesser durchtreten können und/oder an den Membranen adsorbiert werden, wobei eine im wesentlichen partikelarme Proteineinschlusskörper-Lösung erhalten wird.

Es wurde nun festgestellt, dass sich mit dem Verfahren der vorliegenden Erfindung unter Verwendung einer Querstrom-Mikrofiltrationsanlage Proteineinschlusskörper reinigen und konzentrieren lassen. Überraschend und neu ist dabei insbesondere, dass die Proteineinschlusskörper auch bei hoher Belastung der Ausgangspräparation mit löslichen und partikulären Bestandteilen (Biomasse) bei einem stabilen Betriebspunkt filtriert werden können, ohne dass die Membran verstopft und/oder die Filtrationsleistung durch Deckschichtbildung nachlässt. Bisher waren die zu erzielenden Biomassekonzentrationen im Retentat niedrig. Bei hohen Biomassekonzentrationen hingegen bewirkt die Verblockung des Retentatkanals eine zunehmende Veränderung des Betriebspunktes während des Filtrationszyklus bzw. über mehrere Betriebszyklen hinweg, wodurch der Retentatfluss sowie der Permeatflux stetig abnimmt. Die erzielbaren Permeatfluxe ermöglichen nach mehreren Zyklen keine wirtschaftlich attraktive Betriebsweise mehr. Die Reinigung der auf diese Weise verblockten Membranen erweist sich als aufwendig und meistens unvollständig.

Bei geringen Biomassebelastungen der Membran hingegen, die zu geringeren Verblockungsproblemen führen, wird insbesondere auf einem technisch relevanten Maßstab eine extrem große Filtrationsfläche benötigt, die die Wirtschaftlichkeit des Querstrom-Mikrofiltrationsschrittes negativ beeinträchtigt.

Deshalb wurde die Querstrom-Mikrofiltration bisher nicht für die Reinigung und Konzentrierung von Proteineinschlusskörper-Präparaten auf einem technisch relevanten Maßstab und bei hohen Biomassekonzentrationen eingesetzt.

Die hierin eingesetzte, modifizierte Cellulosehydrat Membrankassette hingegen ermöglicht überraschenderweise die Filtration von Proteineinschlusskörper auch bei hoher Belastung der Ausgangspräparation mit löslichen und partikulären Bestandteilen (Biomasse) bei einem stabilen Betriebspunkt, ohne dass die Membran verstopft und/oder die Filtrationsleistung durch Deckschichtbildung nachlässt. Der stabile Betriebspunkt wird überraschenderweise auch über viele Filtrationszyklen erhalten

Mit dem Verfahren der vorliegenden Erfindung werden Proteineinschlusskörper bei hohen Biomassekonzentrationen zwischen 50 g/L und 2500 g/L, bevorzugt zwischen 500 g/L und 1500 g/L, gereinigt und/oder konzentriert. Die Wahl der Abscheidegrenze (cut-off) der Membran hängt dabei ab vom mittleren Partikeldurchmesser und der Partikelgrößenverteilung der Proteineinschlusskörper. Die Abscheidegrenze kann bei der erfindungsgemäß eingesetzten, modifizierten, hydrophilen Cellulosehydrat-Membran zwischen 0.1 bis 0.65 μm, bevorzugt bei 0.45 μm liegen.

Jede Volumengröße kann prozessiert werden, wobei vorzugsweise eine Lösung mit einem Volumen von 1 bis 100.000 L, besonders bevorzugt mit 1-2000 L aufgearbeitet wird. Die Lösung, die die Proteineinschlusskörper enthält, wird unter geeigneten Druckbedingungen an der oder den Membranen vorbeigeleitet, wobei der Überströmdruck vorzugsweise größer ist als der Transmembrandruck (TMP). Bevorzugt wird bei einem TMP von 0.05 bis 3 bar, besonders bevorzugt bei einem TMP von 0.1 bis 0.5 bar, am meisten bevorzugt bei einem TMP von 0.2 bis 0.4 bar, gearbeitet, wobei der Überströmdruck größer ist als der Transmembrandruck. Der Retentatfluss kann über einen weiten Bereich variiert werden und sollte derart gewählt werden, dass eine turbulente Strömung erreicht wird (Reynoldszahl >30; Meyeroltmanns F (1991): BioTec 5: 918-921). Zur Erniedrigung desjenigen, minimalen Retentatflusses, bei dem eine turbulente Strömung erreicht wird, sind in den erfindungsgemäß eingesetzten Membranmodulen Strömungsbrecher eingebaut. Deren Form und geometrische Anordnung ist in einer separaten Patentanmeldung mit dem Titel "Crossflow-Filterkassetten in Form von verbesserten Weitspaltmodulen" eingereicht am gleichen Tag. (WO-A-0185316)

Bei der Biomassebelastung muss unterschieden werden zwischen der Biomassekonzentration im Retentat (angegeben in g/L) und der flächenspezifischen Biomassebelastung (angegeben in $kg/m^2$). Eine zu hohe Biomassekonzentration im Retentat kann zu Verstopfung von Retentatkanälen führen, wogegen eine zu große flächenspezifische Biomassebelastung zu einer Deckschichtbildung auf der Membran (sog. "Fouling") führt.

Die Biomassekonzentration im Retentat der hierin eingesetzten, modifizierten Cellulosehydrat-Module kann bis zu 1500 g/L betragen, während die flächenspezifische Biomassebelastung auf bis zu 30 $kg/m^2$ gesteigert werden kann.

Das Verfahren kann auch bei variierenden Temperaturen durchgeführt werden. Vorzugsweise wird in einem Temperaturbereich von 4°C bis 40°C gearbeitet.

In einem weiteren Aspekt der Erfindung zeigt die erfindungsgemäß gereinigte Proteineinschlusskörper-Präparation eine signifikant geringere Endotoxin-Belastung im Vergleich zum Ausgangsmaterial.

**Beispiele**

In den beschriebenen Versuchen wurden verschiedenartig modifizierte "Screen-Channel"-Module, die mit einer Hydrosart®-Membran aus modifiziertem Cellulosehydrat ausgestattet sind, eingesetzt (Sartorius AG, Deutschland). Dabei unterscheiden sich die Module maßgeblich in der geometrischen Gestaltung des Gewebes. Die Hydrosart®-Membran ist mit Ausschlussgrenzen von 0.1, 0.2, 0.45 sowie 0.65 µm kommerziell erhältlich.

Tabelle 1

| Kenndaten der eingesetzten modifizierten Hydrosart-Module | | | | |
|---|---|---|---|---|
| Modul Lot Nr. (Sartorius AG) | Modultyp-Bezeichnung | Modulart Filterfläche | Geometrische Angaben zum Retentatkanal | Angaben zum Strömungsbrecher ("Screen") |
| 98025101 | #1 | Slice-Modul<br><br>0.1 $m^2$ | Abstand Membran/ Gewebe: 125 µm<br><br>Gewebedicke: 470 µm<br><br>Fadendicke: 210 µm<br><br>Fadenabstand: 250 µm<br><br>Winkel zur Strömungsrichtung: 30° & 60° | Maschengewebe |

Tabelle 1   (fortgesetzt)

| Kenndaten der eingesetzten modifizierten Hydrosart-Module | | | | |
|---|---|---|---|---|
| **Modul Lot Nr. (Sartorius AG)** | **Modultyp-Bezeichnung** | **Modulart Filterfläche** | **Geometrische Angaben zum Retentatkanal** | **Angaben zum Strömungsbrecher ("Screen")** |
| 98055101 | #2 | Slice-Modul<br><br>0.05 m² | Abstand Membran/ Gewebe: 80 µm<br><br>Gewebedicke: 1030 µm<br><br>Fadendicke: 500-650 µm<br><br>Fadenabstand: 1500 µm<br><br>Winkel zur Strömungsrichtung: 45° | Extrudiertes, planares Netz |
| 99015111 | #3 | Slice-Modul<br><br>0.1 m² | Abstand Membran/ Gewebe: 80 µm<br><br>Gewebedicke: 760 µm<br><br>Fadendicke: 340-450 µm<br><br>Fadenabstand: 2400 µm<br><br>Winkel zur Strömungsrichtung: 45° | extrudiertes, planares Netz |
| 99015012 | #3 | Sartocon-2/3-Modul<br><br>0.46 m² | Abstand Membran/ Gewebe: 80 µm<br><br>Gewebedicke: 760 µm<br><br>Fadendicke: 340-450 µm<br><br>Fadenabstand: 2400 µm<br><br>Winkel zur Strömungsrichtung: 45° | Extrudiertes, planares Netz |

## 1. Herstellung einer Ausgangslösung

*E. coli* W3110 Zellmasse, in der ein Zielprotein als Proteineinschlusskörper vorlag (beispielsweise Interleukin-4 R121D Y124D), wurde nach dem Stand der Technik fermentativ hergestellt (Riesenberg et al., 1990: Appl. Microbiol. Biotechnol. 34: 77-82). Die Zellen wurden bei einer Biofeuchtmasse-Konzentration von 30-40% nach enzymatischer Lyse (Lysozym: 1 mg/g Zelltrockengewicht) mit Hilfe eines Standard-Homogenisators (Bran & Lübbe, Deutschland) aufgeschlossen. Als Puffer wurde 100 mM Tris-HCl (pH 8) mit 5 mM Ethylendiamintetraessigsäure (EDTA) verwendet. Der mechanische Zellaufschluss wurde in drei Homogenisationszyklen bei 500 bar erreicht. Der Aufschlussgrad wurde bildanalytisch zu ca. 80-90% bestimmt. Das gewonnene Rohhomogenat wurde anschließend mit Hilfe eines Separators (Westfalia, Deutschland) konzentriert, wobei die gesamte Trockenmasse im Schlamm aufgefangen wurde. Das fließfähige Sediment wurde anschließend in flüssigem Stickstoff cryopelletiert und bis zur weiteren Verwendung für die Filtrationsexperimente bei -70°C gelagert.

Mit Hilfe dieses Verfahrens wurden drei unabhängige Rohhomogenat-Chargen hergestellt und als Cryopellets bei -70°C bis zur weiteren Verwendung gelagert.

## 2. Messung der Endotoxin-Abreicherung nach Querstrom- Mikrofiltration

Proben der Proteineinschlusskörper-haltigen Lösungen (Retentat vor der Diafiltration, Retentat nach der Diafiltration) sowie das Gesamtpermeat werden mit Hilfe des Limulus-Amöbozyten-Lysat-Verfahrens nach der Europäischen Pharmacopoe (Pharm. Eur.) analysiert. Die Prüfung auf Endotoxine erfolgte nach der "Festgel"-Methode, in der die Zugabe einer endotoxinhaltigen Lösung zu einer Limulus-Amöbozyten-Lysat-Lösung (LAL-Lösung) eine Gelbildung eines Gemisches verursacht. Der Gelbildung liegt eine in mehreren Schritten ablaufende Gerinnungskaskade zugrunde.

## 3. Querstrom-Mikrofiltration mit Standard-Hydrosart®-Modul

### Anlagenaufbau:

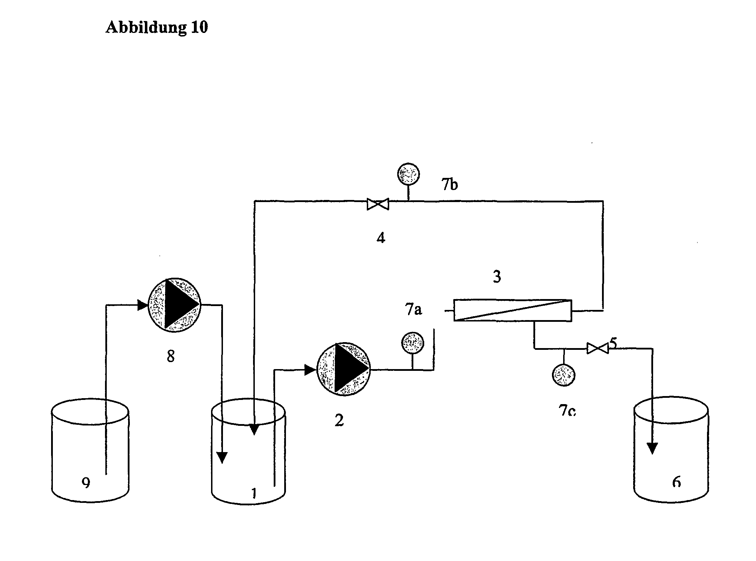

Eine Querstrom-Mikrofiltrationsanlage besteht typischerweise den Komponenten, die in **Abbildung 10** dargestellt sind.

### Bestimmung der Wasserwerte:

Die Anlage wird zur Wasserwertbestimmung mit vollentsalztem Wasser (VE-Wasser) gefahren, wobei die folgenden Drücke mit Hilfe der Drosselventile (4 & 5) eingestellt werden:

R(in):      2 bar
R(out):     1 bar
P:          0.5 bar
TMP:        1 bar

Am Retentatausgang und Permeatausgang werden jeweils die Flüsse bestimmt. Die Messung dient dazu, den Zustand der Kassetten vor der ersten Verwendung und vor bzw. nach jedem Produktzyklus zu bestimmen.

Anschließend wird die gesamte Anlage mit Waschpuffer (0.05 M Tris-HCl pH 7, 5 mM Ethylendiamintetraacetat-$Na_4$, 0.1% Zwittergent 3-14) hinreichend gespült. Das Drosselventil für den Permeatausgang (5) wird geschlossen und die Pumpe wird gestoppt. Die Retentatvorlage wird entleert und anschließend mit Proteineinschlusskörper-Suspension befüllt.

### Einspülen von Rohhomogenat und Anfahren der Anlage

Ein Teil des cryopelletierten Rohhomogenats, der z.B 300 g ursprünglichem Zellfeuchtgewicht entspricht, wird aufgetaut und in ein separates Retentatvorlagegefäß vorgelegt. Die Suspension wird mit Waschpuffer (0.05 M Tris-HCl pH 7, 5 mM Ethylendiamintetraacetat-$Na_4$, 0.1% Zwittergent 3-14 (Fluka)) auf das angegebene Gesamt-Retentatvolumen abzüglich Totvolumen aufgefüllt und an den Pumpeneingang angeschlossen. Das Totvolumen der Anlage ist 0.55 L. Die Pumpe wird bei geschlossenem Permeat-Drosselventil (5) gestartet und das Retentat wird ca. 5 Minuten im Kreis gepumpt. Dabei werden die folgenden Drücke durch Adjustierung der Pumpleistung (R(in)) bzw. mit Hilfe der Drosselventile eingestellt:

R(in):      1 bar
R(out):     0.5 bar
P:          0.4 bar
TMP:        0.35 bar

Das Permeatventil wird dabei besonders vorsichtig geöffnet, um die Komprimierung einer möglicherweise auf der Membran vorhandenen Deckschicht zu vermeiden. Aus den angegebenen Einstellungen errechnet sich ein Transmembrandruck (TMP) von 0.35 bar, der sich in zuvor durchgeführten Betriebspunktbestimmungen als optimal herausstellte.

*Diafiltration*

Während der nun folgenden Diafiltration wird das Retentatvolumen bis zu fünf mal ausgetauscht. Das Retentatvolumen wird durch kontinuierliche Zugabe von Waschpuffer auf einem konstanten Niveau gehalten.

Nach jeweils 0.5 L Permeatvolumen werden die Druckeinstellungen überprüft und dokumentiert (konstanter TMP von 0.35 bar) und Proben aus Retentat und Permeat gezogen. Ferner werden der Retentat- sowie der Permeatfluss bestimmt.

*Konzentrierung*

Am Ende der Diafiltration kann optional das Retentat durch Unterbrechung der Waschpufferzufuhr auf etwa 50% des ursprünglichen Volumens konzentriert werden.

*Entnahme des Retentats*

Zur Entnahme des Retentats wird das Permeat-Drosselventil (5) langsam geschlossen und das Retentat im Kreis gepumpt. Anschließend werden 0.5-1 L Waschpuffer (=1 bis 2 x Totvolumen) in die Anlage hineingefahren und dabei das Retentat in die Retentatvorlage vollständig entleert.

*Reinigung der Anlage*

Zunächst wird mit 2 L 0.9% NaCl-Lösung die Anlage gespült, wobei das Permeat-Drosselventil (5) geschlossen bleibt. Die Pumpleistung wird so eingestellt, dass der Druck am Retentateingang (R(in)) 3 bar beträgt. Das Drosselventil am R(out) (7b) wird vollständig geöffnet. Dann werden weitere 2 L 0.9% NaCl-Lösung ca. 10 Minuten im Retentatkreislauf gepumpt.

Anschließend wird die Anlage mit 2.5 L 2%iger Citronensäure-Lösung gespült. Die Druckeinstellungen bleiben gleich. Die ersten 0.5 L werden aus der Anlage ausgetragen, anschließend wird die Lösung ca. 10 Minuten im Retentatkreislauf gefahren.

Die Citronensäure-Reinigungslösung wird mit ca. 20 L VE-Wasser ausgespült. Anschließend werden 2.5 L 1%ige Ultrasil-62-Lösung (Henkel AG, Deutschland), die auf ca. 40°C temperiert ist, eingespült. Die Druckeinstellungen bleiben gleich. Die ersten 0.5 L werden aus der Anlage ausgetragen, anschließend wird die Lösung ca. 10 Minuten im Retentatkreislauf gefahren.Die Ultrasil-Reinigungslösung wird wieder mit ca. 20 L VE-Wasser ausgespült.

Abschließend erfolgt eine Reinigung mit 2.5 L einer 1 N NaOH-Lösung, die auf 40°C temperiert ist. Die Druckeinstellungen bleiben gleich. Die ersten 0.5 L werden aus der Anlage ausgetragen, anschließend wird die Lösung ca. 10 Minuten im Retentatkreislauf gefahren. Dann wird das Drosselventil am Permeatausgang (5) vollständig geöffnet. Nachfolgend wird die Anlage mit ca. 20 L VE-Wasser von Natronlauge freigespült.

Zum Schluss erfolgt erneut eine Wasserwertbestimmung (s.oben).

*Lagerung der Kassetten*

Kurzzeitig (bis zu 7 Tagen) kann die gesamte Anlage inkl. der eingebauten Kassetten in VE-Wasser gelagert werden. Bei längerer Standzeit (>7 Tage) wird die gesamte Anlage inkl. der eingebauten Kassetten in 20% wässrigem Ethanol gelagert.

*In-Prozess-Monitoring*

Die Proben werden abzentrifugiert (14.000 x g, 15 Minuten) und mit Waschpuffer geeignet verdünnt. Anschließend wird die OD bei 280 nm in einem handelsüblichen Spektralphotometer gegen eine Referenzküvette (Waschpuffer) vermessen.

Unter Verwendung des beschriebenen Versuchsprotokolls wurden ausgehend von einer einheitlichen Rohhomogenatpartie (s. Beispiel 1) mehrere Filtrationszyklen gefahren.

*Ergebnisse*

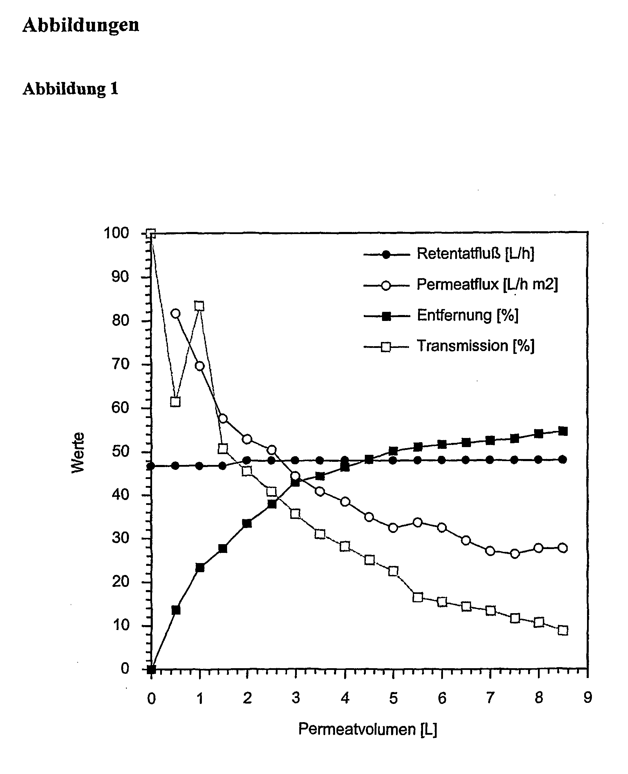

Die Resultate des ersten Diafiltrationszyklus (neue Hydrosart-Slice-Membran (0.1 m$^2$, 0.45 µm)) von *E. coli* Rohhomogenat (Partie #1) mit Proteineinschlusskörpern von Interleukin-4 R121D Y124D sind in **Abbildung 1** zusammengestellt.

Die Entfernung von löslichen Bestandteilen aus dem Retentat nimmt mit der Filtrationsdauer zu und endet nach 3.3 Volumenaustauschen bei einem Plateauwert von 55 %. Die Transmission nimmt ausgehend von 100%, was einer vollkommen freien Membran entspricht, stetig ab auf einen Endwert von 9 % nach 3.3 Volumenaustauschen. Der Retentatfluss liegt weitgehend konstant bei 46-48 L/h. Der Permeatflux nimmt stetig ab von 82 L/h m$^2$ auf 27 L/h m$^2$ nach 3.3 Volumenaustauschen.

Vor der Produktfahrt wurden die Wasserwerte zu 37.2 L/h (Retentatfluss) bzw. 1968 L/h m$^2$ (Permeatflux) des neuen Membranmoduls bestimmt. Nach der Produktfahrt und Reinigung des Moduls (s.o.) wurden nur noch 22.8 L/h (Retentatfluss) bzw. 1800 L/h m$^2$ (Permeatflux) gemessen.

In dem unmittelbar nächsten Produktzyklus wurden exakt die gleichen Ausgangsbedingungen hergestellt und das gleiche Ausgangsprodukt verwendet. Die Eingangswasserwerte lagen bei 22.8 L/h (Retentatfluss) bzw. 1848 L/h m$^2$ (Permeatflux). Nach Einspülen des Rohhomogenats war bereits nach 1.5 L Permeatvolumen der Retentatfluss auf 2.5 L/h und der Permeatflux auf 2 L/h m$^2$ zusammengebrochen. Die Produktfahrt wurde daraufhin abgebrochen und die Anlage nach obigem Protokoll wieder in den Ausgangszustand versetzt. Die Wasserwerte nach Reinigung der Anlage lagen bei 19.4 L/h (Retentatfluss) bzw. 1704 L/h m$^2$.

In dem unmittelbar folgenden Produktzyklus wurden wiederum exakt die gleichen Ausgangsbedingungen hergestellt (s.o.) und das gleiche Ausgangsprodukt eingesetzt. Die Wasserwerte vor der Produktfahrt lagen bei 19.6 L/h (Retentatfluss) bzw. 1680 L/h m$^2$ (Permeatflux). Die Rohhomogenat-Suspension ließ sich bei diesem Zyklus aber nicht mehr in die Anlage einspülen. Der Retentatkanal war sofort verblockt. Die Produktfahrt wurde daraufhin abgebrochen und die Anlage nach obigem Protokoll wieder in den Ausgangszustand versetzt. Die Kassette war mit dem oben beschriebenen Standard-Reinigungsprotokoll nicht mehr zu reinigen.

### *Schlussfolgerung*

Anhand der angegebenen Wasserwerte für Retentatfluss und Permeatflux sowie anhand der genannten Retentatflüsse und Permeatfluxe bei Produktfahrt ist ersichtlich, dass der Retentatkanal bei 230 g Zellfeuchtgewicht-Äquivalent/L durch Ablagerung von Partikeln verblockte. Auch die Membran selbst wurde über bereits drei Zyklen durch Deckschichtbildung bei einer Biomassebelastung der Membran von 300 g Zellfeuchtgewicht/m$^2$ signifikant verblockt.

### 4. Querstrom-Mikrofiltration mit modifizierten Hydrosart®-Modulen

Die experimentelle Anordnung, Vorgehensweise sowie die Reinigungsvorschriften wurden analog zu Beispiel 1 gewählt. Das Herstellung des Ausgangsmaterials (s. Beispiel 1) sowie die Biomassebelastung der Membran (3 kg Zellfeuchtgewicht-Äquivalent/m$^2$) und die Biomassekonzentration (230 g Zellfeuchtgewicht-Äquivalent/L) waren identisch zu den in Beispiel 2 genannten Bedingungen.

Es wurden drei modifizierte Hydrosart-Module mit unterschiedlich ausgeführten Strömungsbrechern (sog. "Screens") und Kanalgeometrien getestet (s. allgemeinen Teil in der Sektion Beispiele). Die Membran selbst wurde dabei nicht variiert (Hydrosart mit einer Ausschlussgrenze von 0.45 μm).

### 4.1 Ergebnisse mit dem Typ#1-Modul

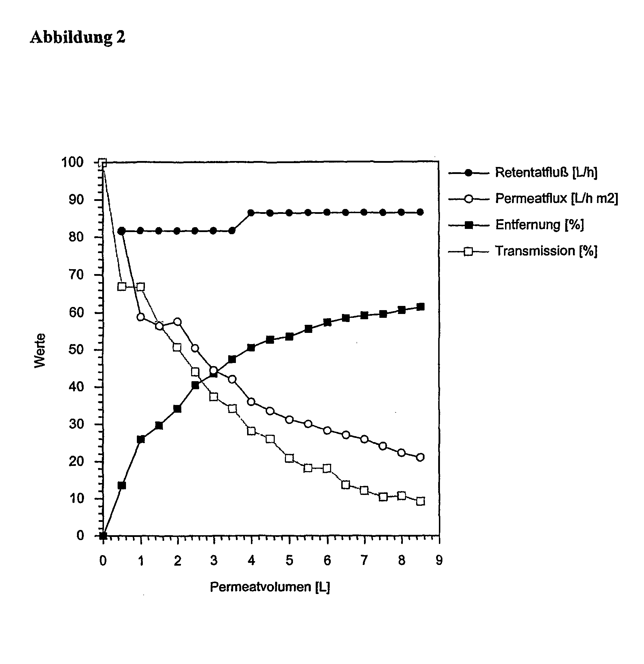

In **Abbildung 2** ist beispielhaft der erste Diafiltrationszyklus (neues Hydrosart-Slice-Modul Typ#1, 0.1 m$^2$, 0.45 μm) von *E. coli* Rohhomogenat (Partie #1) mit Proteineinschlusskörpern von Interleukin-4 R121D Y124D veranschaulicht. Die Entfernung von löslichen Bestandteilen aus dem Retentat nimmt mit der Filtrationsdauer zu und endet nach 3.3 Volumenaustauschen bei einem Plateauwert von 61%. Die Transmission nimmt ausgehend von 100%, was einer vollkommen freien Membran entspricht, stetig ab auf einen Endwert von 9% nach 3.3 Volumenaustauschen. Der Retentatfluss liegt weitgehend konstant bei 81-86 L/h. Der Permeatflux nimmt stetig ab von 82 L/h m$^2$ auf 21 L/h m$^2$ nach 3.3 Volumenaustauschen.

Vor der Produktfahrt wurden die Wasserwerte zu 52.8 L/h (Retentatfluss) bzw. 2136 L/h m$^2$ (Permeatflux) des fabrikneuen, modifizierten Membranmoduls bestimmt. Nach der Produktfahrt und Reinigung des Moduls (s.o.) wurden nur noch 46.8 L/h (Retentatfluss) bzw. 1968 L/h m$^2$ (Permeatflux) gemessen.

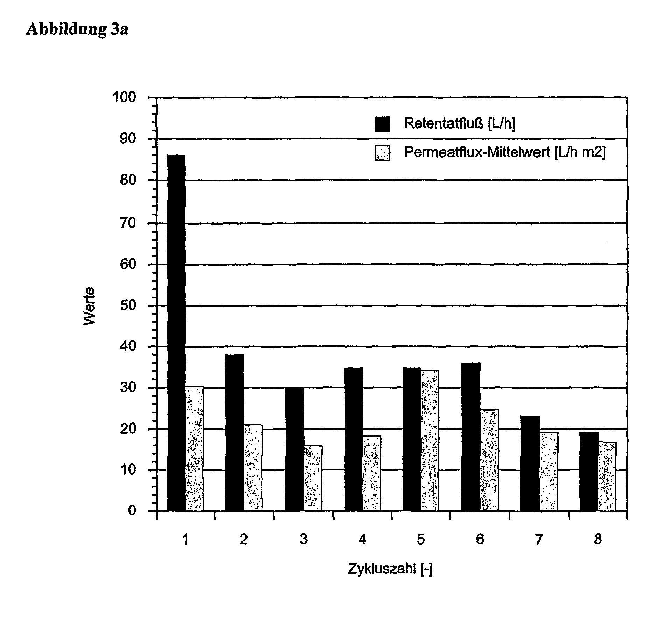

Die Retentatflüsse und Permeatfluxe bei Produktfahrt der folgenden 7 Produktfahrten sind in **Abbildung 3a** zusammengefasst. Der Retentatfluss bei Produktfahrt nimmt von 86 L/h im ersten Zyklus auf 19 L/h im achten Zyklus ab. Der größte Einbruch im Retentatfluss bei Produktfahrt wurde zwischen dem ersten und dem zweiten Zyklus beobachtet. Der mittlere Permeatflux schwankt zwischen 15 und 35 L/h m$^2$.

Die Entfernung von löslichen Bestandteilen aus dem Retentat liegt bei allen Produktfahrten zwischen 60% und 80%. Die mittlere Transmission liegt zwischen 40% und 80%.

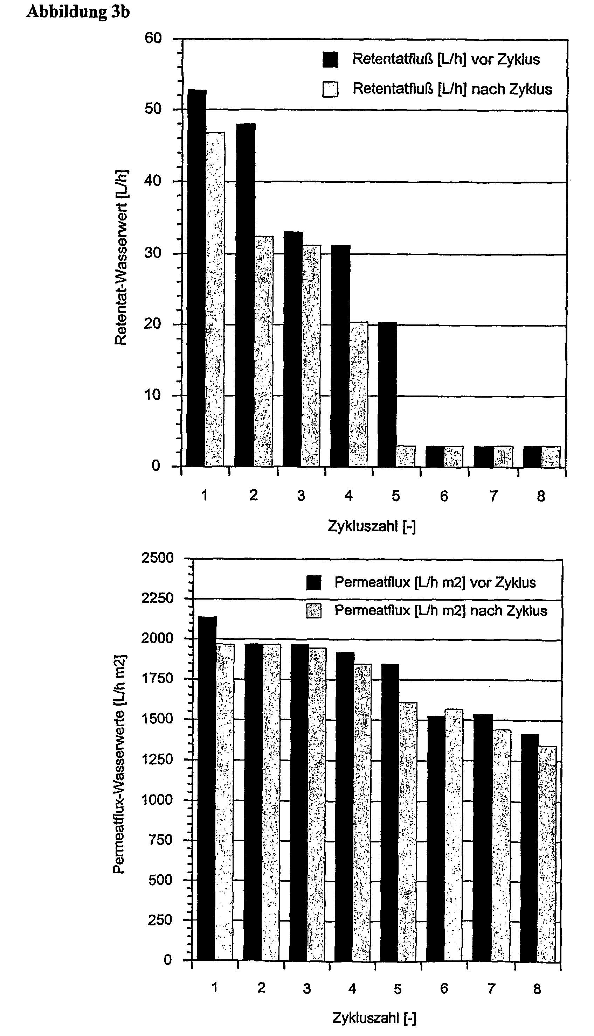

Die Ergebnisse der Reinigung des Hydrosart-Typ#1-Moduls, d.h. die Wasserwerte vor und nach Produktfahrt für Retentatfluss und Permeatflux, sind in **Abbildung 3b** dargestellt. Es ist deutlich zu erkennen, dass der Retentat-Wasserwert über die Zyklen stetig abnimmt und beim 5 Zyklus praktisch zusammenbricht. Dies deutet, wie bei der Standard-Hydrosart-Kassette, auf ein Verblockungsproblem beim Retentateingang hin.

Die Wasserwerte für den Permeatflux fallen innerhalb von 8 Zyklen von 2136 L/h m$^2$ auf 1344 L/h m$^2$, d.h. um 37%, ab. Die Effizienz des einzelnen Reinigungsschrittes liegt zwischen 87% (schlechteste Schritteffizienz) und 100% (beste Schritteffizienz).

*Schlussfolgerung*

Die modifizierte Hydrosart-Kassette Typ#1 zeigt bereits deutlich verbesserte Leistungsdaten und konnte über acht Produktfahrten hinweg eingesetzt werden, obschon unter gleichen Bedingungen die Standard-Hydrosart-Kassette bereits nach drei Produktfahrten irreversibel verblockt war.

Dennoch zeigt sich bei der im Typ#1 verwendeten Kanalgeometrie und Struktur der Strömungsbrecher ("Screen") nach wie vor das Problem der Verblockung des Retentatkanals über die Zyklen. Durch die stetigen Veränderungen innerhalb eines Produktzyklus und über mehrere Zyklen hinweg ist auch mit dem Typ#1-Modul kein stabiler Betriebspunkt einzuhalten.

### 4.2 Ergebnisse mit dem Typ#2-Modul

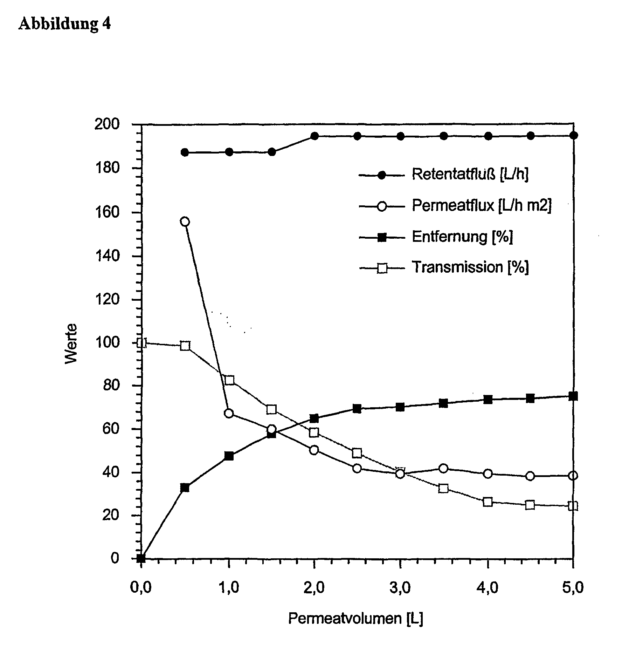

In **Abbildung 4** ist beispielhaft der erste Diafiltrationszyklus (neues Hydrosart-Slice-Modul Typ#2, 0.05 m$^2$, 0.45 μm) von E. coli Rohhomogenat (Partie #1) mit Proteineinschlusskörpern von Interleukin-4 R121D Y124D veranschaulicht. Die Entfernung von löslichen Bestandteilen aus dem Retentat nimmt mit der Filtrationsdauer zu und endet nach 3.8 Volumenaustauschen bei einem Plateauwert von 75%. Die Transmission nimmt ausgehend von 100%, was einer vollkommen freien Membran entspricht, stetig ab auf einen Endwert von 25 % nach 3.8 Volumenaustauschen. Die mittlere Transmission liegt bei 53%. Der Retentatfluss liegt weitgehend konstant bei 190 L/h. Der Permeatflux nimmt, ausgehend von einem Startwert von 156 L/h m$^2$, stetig ab und endet bei einem Wert von 38 L/h m$^2$.

Vor der Produktfahrt wurden die Wasserwerte zu 648 L/h (Retentatfluss) bzw. 3744 L/h m$^2$ (Permeatflux) des fabrikneuen, modifizierten Membranmoduls bestimmt. Nach der Produktfahrt und Reinigung des Moduls (s.o.) wurden 708 L/h (Retentatfluss) bzw. 3720 L/h m$^2$ (Permeatflux) gemessen.

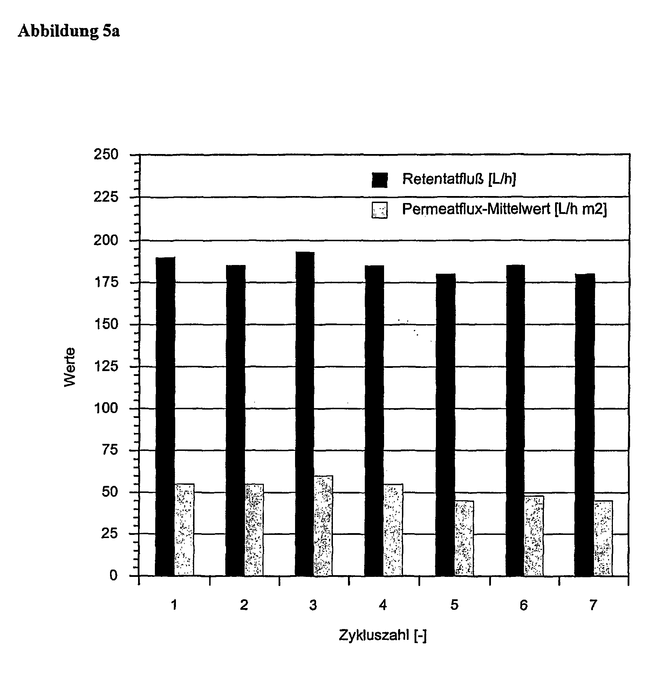

Die Retentatflüsse und Permeatfluxe bei Produktfahrt der folgenden 6 Produktfahrten sind in **Abbildung 5a** zusammengefasst. Der Retentatfluss bei Produktfahrt ist weitgehend konstant und schwankt um 185 L/h. Es wird kein Einbruch im Retentatfluss bei Produktfahrt beobachtet. Der mittlere Permeatflux schwankt um 50 L/h m$^2$.

Die Entfernung von löslichen Bestandteilen aus dem Retentat liegt bei allen Produktfahrten zwischen 60% und 80%. Die mittlere Transmission liegt zwischen 45% und 75%.

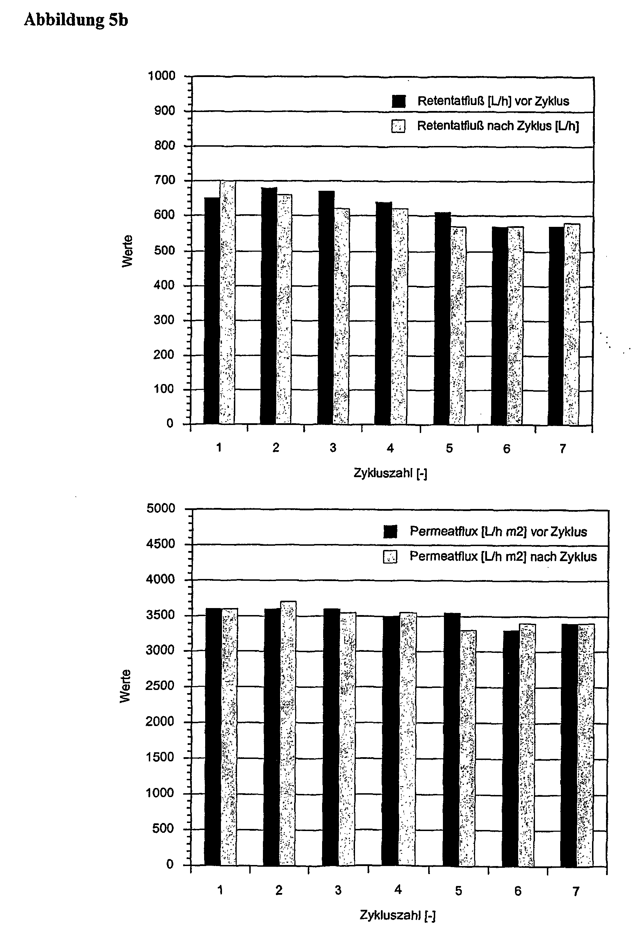

Die Ergebnisse der Reinigung des Hydrosart-Typ#2-Moduls, d.h. die Wasserwerte vor und nach Produktfahrt für Retentatfluss und Permeatflux, sind in **Abbildung 5b** dargestellt. Die Wasserwerte für den Retentatfluss sind leicht abnehmend und liegen zwischen 650-700 L/h im 1. Zyklus und 580 L/h im letzten Zyklus. Dies deutet darauf hin, dass das Verblockungsproblem beim Retentateingang im Vergleich zur Standard-Hydrosart-Kassette bzw. zum modifizierten Hydrosart-Typ#1-Modul deutlich vermindert ist.

Die Wasserwerte für den Permeatflux fallen innerhalb von 7 Zyklen nur leicht von 3744 L/h m$^2$ auf 3408 L/h m$^2$, d.h. um nur 9%, ab. Die Effizienz des einzelnen Reinigungsschrittes liegt zwischen 92% (schlechteste Schritteffizienz) und 105% (beste Schritteffizienz).

*Schlussfolgerung*

Die modifizierte Hydrosart-Kassette Typ#2 zeigt gegenüber dem Standard-Hydrosart-Modul und gegenüber dem Hydrosart-Typ#1-Modul deutlich verbesserte Leistungsdaten und konnte über sieben Produktfahrten hinweg eingesetzt werden, obschon unter gleichen Bedingungen die Standard-Hydrosart-Kassette bereits nach drei Produktfahrten irreversibel verblockt war.

Bei der im Hydrosart-Typ#2-Modul verwendeten Kanalgeometrie und Struktur der Strömungsbrecher ("Screen") ist das Problem der Verblockung des Retentatkanals über die Zyklen signifikant reduziert. Durch die konstanten Bedingungen innerhalb eines Produktzyklus und über mehrere Zyklen hinweg ist mit dem Hydrosart-Typ#2-Modul ein stabiler Betriebspunkt auch bei einer Biomassebelastung von 3 kg Zellfeuchtgewicht-Äquivalent/m$^2$ (230 g Zellfeuchtgewicht-Äquivalent/L) einzuhalten.

### 4.3 Ergebnisse mit dem Typ#3-Modul

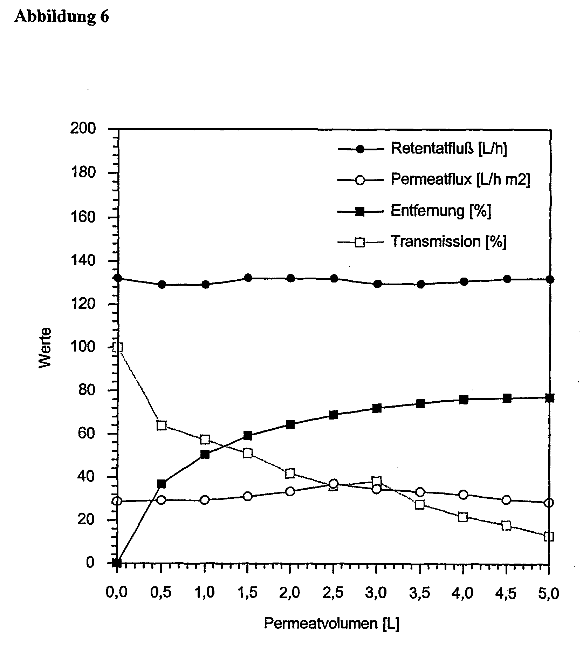

In Abbildung 6 ist beispielhaft der erste Diafiltrationszyklus (neues Hydrosart-Slice-Modul Typ#3, 0.1 m$^2$, 0.45 m) von *E. coli* Rohhomogenat (Partie #2) mit Proteineinschlusskörpern von Interleukin-4 R121D Y124D veranschaulicht. Das Retentatvolumen wurde bei diesen Versuchen auf 1.3 L gesenkt (0.55 L Totvolumen). Die Entfernung von löslichen Bestandteilen aus dem Retentat nimmt mit der Filtrationsdauer zu und endet nach bereits 3.8 Volumenaustauschen bei einem Plateauwert von 77%. Die Transmission nimmt ausgehend von 100%, was einer vollkommen freien Membran entspricht, stetig ab auf einen Endwert von 13% nach 3.8 Volumenaustauschen. Der Retentatfluss liegt weitgehend konstant bei 129-132 L/h. Der Permeatflux ist praktisch konstant bei einem Mittelwert von 31.7 L/h m$^2$ (±2.95).

Vor der Produktfahrt wurden die Wasserwerte zu 105.6 L/h (Retentatfluss) bzw. 1320 L/h m$^2$ (Permeatflux) des fabrikneuen, modifizierten Membranmoduls bestimmt. Nach der Produktfahrt und Reinigung des Moduls (s.o.) wurden 110.4 L/h (Retentatfluss) bzw. 1344 L/h m$^2$ (Permeatflux) gemessen.

Parallel wurde ein Experiment mit einer Durapore V-Screen Kassette (Ausschlussgrenze 0.45 μm, 0.1 m$^2$ Filterfläche, hydrophilisiertes Polyvinylidenfluorid (PVDF), Modul ohne Strömungsbrecher (engl. "open channel")) unter gleichen Bedingungen durchgeführt. Dabei wurde die gleiche Versuchsanordnung gewählt, wie unter Beispiel 2 beschrieben. Der Kassettenhalter wurde allerdings gegen einen entsprechenden Halter für die Durapore-Membran ausgetauscht. Die Biomassebelastung (Rohhomogenat Partie #2) lag in diesem Experiment bei ebenfalls 3 kg Zellfeuchtgewicht-Äquivalent/m$^2$ bzw. 230 g Zellfeuchtgewicht-Äquivalent/L. Das Retentatvolumen betrug 1.3 L und es wurden 3.8 Volumenaustausche durchgeführt (5 L Permeatvolumen). Der Retentatfluss betrug 90 L/h. Der initale TMP wurde mit 0.3 bar festgelegt. In diesem Experiment wurde allerdings nicht, wie bei allen übrigen Versuchen, der TMP konstant gehalten, sondern der Permeatflux wurde auf einen Wert von 38 L/h m$^2$ fixiert. Ein Anstieg im TMP zeigt gleichermaßen die Verblockung von Membranporen an, wie dies in den anderen Versuchen der Abfall an Permeatflux anzeigt.

Nach 3.8 Volumenaustauschen wurde eine Entfernung von 70% erhalten. Die Transmission fiel von inital 100% auf 10% ab. Der TMP stieg während der Filtration von 0.3 bar auf 0.65 bar an. Der Retentatfluss wurde in diesem Experiment nicht verfolgt.

*Schlussfolgerung*

Das Hydrosart-Typ#3-Modul zeigt unter gleichen Bedingungen gegenüber dem Durapore-V-Screen Modul die besseren Leistungsdaten. Es werden beim Hydrosart-Typ#3-Modul ca. 80% des löslichen Proteinanteils aus dem Retentat abgereichert, während beim Durapore-V-Screen-Modul etwa 70% Entfernung erreicht wird. Die Transmissionsverläufe sind in beiden Fällen vergleichbar. Vor allem aber der Permeatflux (bzw. der TMP) ist beim Hydrosart-Typ#3-Modul annähernd konstant, während der TMP im Falle des Durapore-V-Screen-Moduls deutlich ansteigt (bzw. der Permeatflux abnimmt), wie dies auch bei den anderen getesteten Modultypen beobachtet wurde.

### 4.3.1 Schrittweise Erhöhung der Biomassebelastung der Membran

Aufgrund der Tatsache, dass bei Verwendung des Hydrosart-Typ#3-Moduls sowohl der Retentatfluss, als auch der Permeatflux über die Filtrationsdauer praktisch konstant verlaufen, wurde die Biomassebelastung der Membran sowie die Biomassekonzentration im Retentat in der folgenden Versuchsserie schrittweise bis zum Faktor 5 erhöht. Dabei wurde eine zweite Rohhomogenat-Partie (#2) eingesetzt.

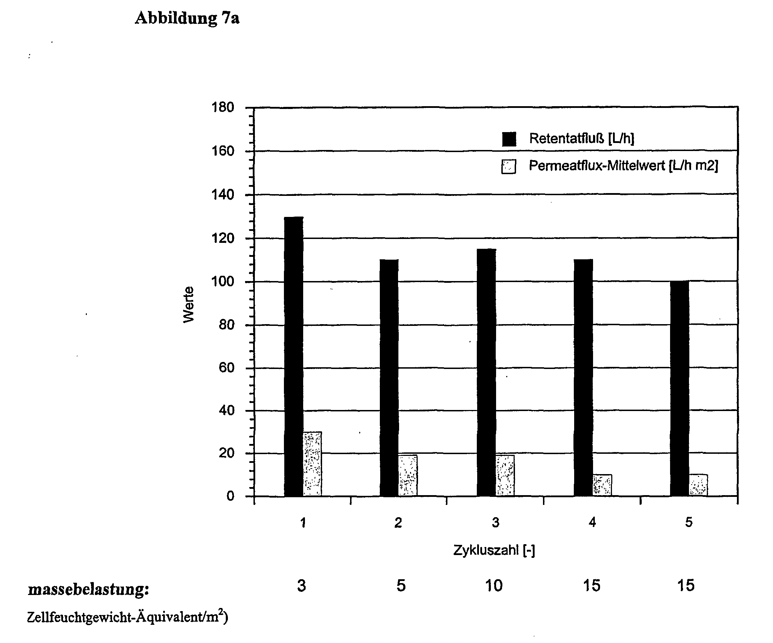

Die Retentatflüsse und Permeatfluxe bei Produktfahrt der folgenden 4 Produktfahrten mit steigenden Biomassebelastungen der Membran sind in **Abbildung 7a** zusammengefasst. Der Retentatfluss bei Produktfahrt nimmt von 130 L/h im ersten Zyklus bei 3 kg Zellfeuchtgewicht-Äquivalent/m$^2$ auf 100 L/h im vierten Zyklus mit 15 kg Zellfeuchtgewicht-Äquivalent/m$^2$ ab. Bei Steigerung der Biomassebelastung um 500% geht der Retentatfluss also nur um 23% zurück. Der mittlere Permeatflux geht von 30 L/h m$^2$ bei 3 kg Zellfeuchtgewicht-Äquivalent/m$^2$ im ersten Zyklus auf 10 L/h m$^2$ bei 15 kg Zellfeuchtgewicht-Äquivalent/m$^2$ im vierten Zyklus zurück. Der Permeatflux erniedrigt sich also um 50% bei Steigerung der Biomassebelastung um 500%.

Die Entfernung von löslichen Bestandteilen aus dem Retentat liegt bei 3 kg Zellfeuchtgewicht-Äquivalent/m$^2$ bei 75% und schwankt bei Steigerung der Biomassebelastung auf 5, 10 und 15 kg Zellfeuchtgewicht-Äquivalent/m$^2$ um 60%. Die mittlere Transmission liegt zwischen 30% und 40%.

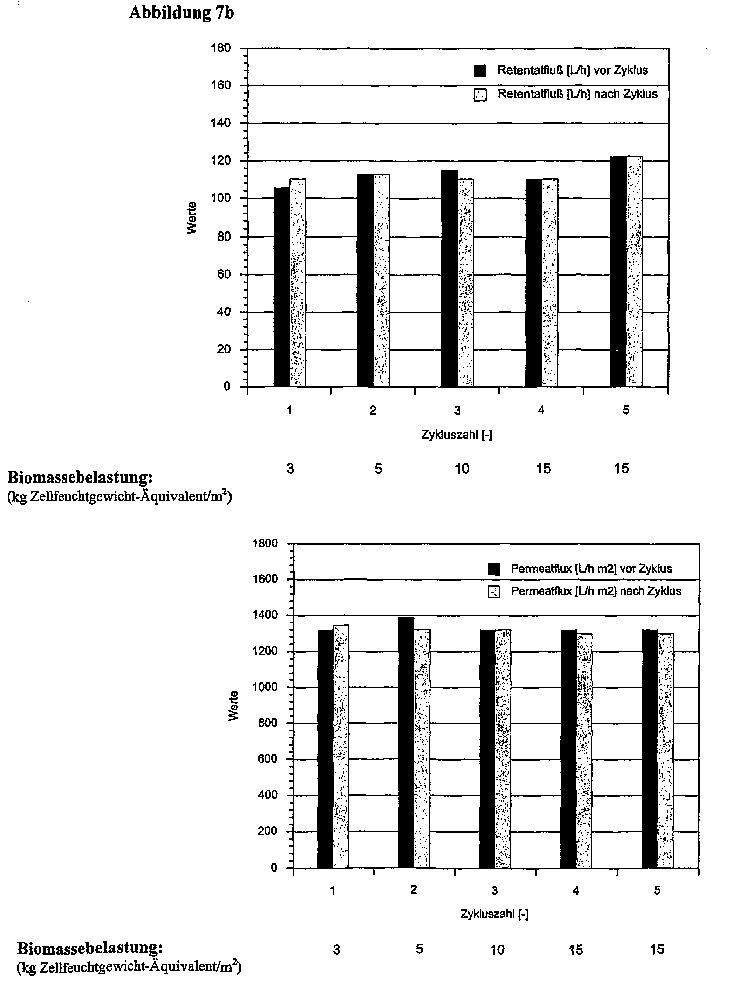

Die Ergebnisse der Reinigung des Hydrosart-Typ#3-Moduls, d.h. die Wasserwerte vor und nach Produktfahrt für Retentatfluss und Permeatflux, sind in **Abbildung 7b** dargestellt. Die Wasserwerte sind sowohl für den Retentatfluss als auch für den Permeatflux weitgehend konstant, obwohl die Biomassebelastung der Membran um 500% erhöht wurde. Dies deutet darauf hin, dass das Verblockungsproblem beim Retentateingang sowie das Verblockungsproblem der Membranporen durch die baulichen Veränderungen am Modul signifikant reduziert wurde.

Die Effizienz des einzelnen Reinigungsschrittes liegt zwischen 93% (schlechteste Schritteffizienz) und 100% (beste Schritteffizienz).

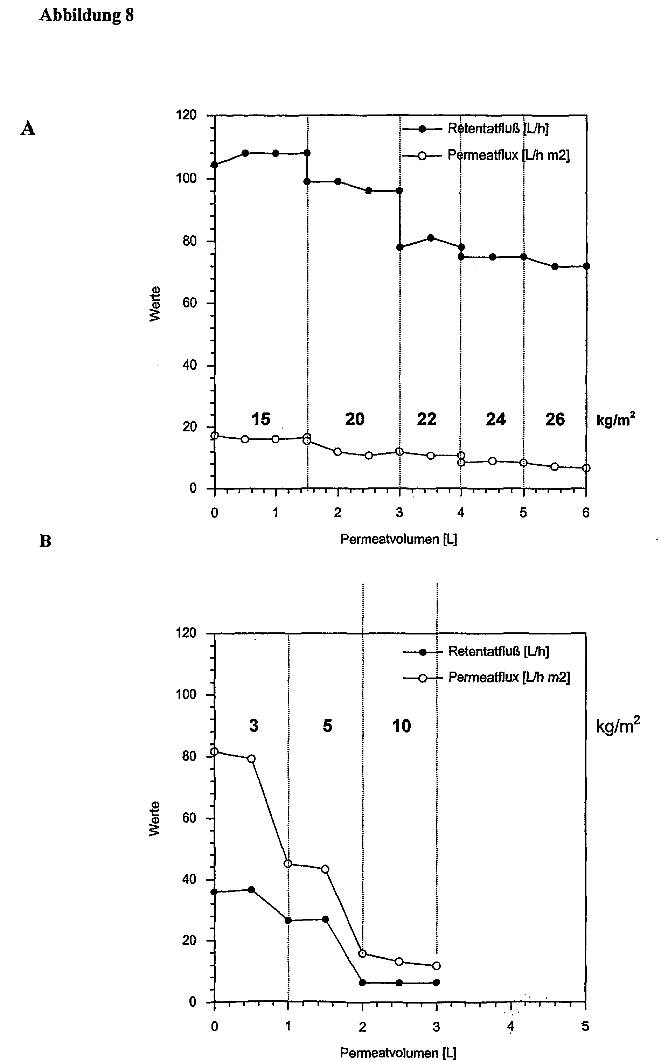

In einem weiteren Experiment wurde die genaue Grenze der Biomassebelastbarkeit des Hydrosart-Typ#3-Moduls ermittelt. Dazu wurde, ausgehend von einer Biomassebelastung von 15 kg Zellfeuchtgewicht-Äquivalent/m$^2$, mit der die Diafiltration gestartet wurde, zu bestimmten Zeitpunkten die Konzentration an Proteineinschlusskörpem im Retentat erhöht. Die Ergebnisse dieses Experiments sind in **Abbildung 8** (A) zusammengefasst. Der Retentatfluss geht bei 50%iger Steigerung der Biomassekonzentration im Retentat von 1153 g Zellfeuchtgewicht-Äquivalent/L auf 1733 g/L von 108 L/h auf 72 L/h, also um 33% zurück. Der Permeatflux sinkt bei 70%iger Steigerung der Biomassebelastung der Membran von 15 kg Zellfeuchtgewicht-Äquivalent/$m^2$ auf 26 kg/$m^2$ von ca. 16 L/h $m^2$ auf ca. 7 L/h $m^2$, also um 56%, ab. Die obere Grenze für die Biomassebelastung der Membran liegt somit bei etwa 22 kg Zellfeuchtgewicht-Äquivalent/$m^2$. Oberhalb dieser Grenze sinkt zum einen der Permeatflux unter einen Wert von 10 L/h $m^2$ ab und zum anderen nimmt der Retentatfluss deutlich weiter ab.

Eine weitere Steigerung der Biomassebelastbarkeit auf 30 kg Zellfeuchtgewicht-Äquivalent/$m^2$, entsprechend 2307 g Zellfeuchtgewicht-Äquivalent/L, im folgenden Zyklus war nicht mehr möglich und führte zur Verblockung des Retentateingangs während des Einspülvorgangs. Das Modul war allerdings nach wie vor vollständig reinigbar und zeigte nach Durchführung des Standard-Reinigungsprotokolls (s. Beispiel 2) einen Retentatwasserwert von 122 L/h und einen Permeatwasserwert von 1320 L/h $m^2$.

In einem Vergleichsversuch mit dem Standard-Hydrosart-Modul (0.1 $m^2$, Slice-Format, Lot Nr. 96108741 / Nr. 008), bei dem das Rohhomogenat Partie #3 eingesetzt wurde, wurde ausgehend von einer Biomassebelastung von 3 kg Zellfeuchtgewicht-Äquivalent/$m^2$ (230 g Zellfeuchtgewicht-Äquivalent/L) die Biomassekonzentration im Retentat stufenweise auf 10 kg Zellfeuchtgewicht-Äquivalent/$m^2$ erhöht **(Abbildung 8 (B))**. Der Retentatfluss sowie der Permeatflux bei Produktfahrt nehmen auch innerhalb einer Biomassekonzentrationsstufe signifikant ab. Bereits bei 10 kg Zellfeuchtgewicht-Äquivalent/$m^2$ (769 g Zellfeuchtgewicht-Äquivalent/L) liegen die Permeatfluxe nur noch bei 12-13 L/h $m^2$, obschon nur wenige Permeatvolumina diafiltriert wurde.

*Schlussfolgerung*

Die modifizierte Hydrosart-Kassette Typ#3 zeigt signifikant verbesserte Leistungsdaten und konnte über fünf Produktfahrten hinweg eingesetzt werden, wobei die Biomassebelastung um 500% gesteigert wurde. Unter gleichen Versuchsbedingungen war die Standard-Hydrosart-Kassette bereits nach drei Produktfahrten bei einer Biomassebelastung von 3 kg Zellfeuchtgewicht-Äquivalent/$m^2$ irreversibel verblockt.

Bei der im Typ#3 verwendeten Kanalgeometrie und Struktur der Strömungsbrecher ("Screen") ist das Problem der Verblockung des Retentatkanals über die Zyklen nicht mehr erkennbar. Durch die konstanten Bedingungen innerhalb eines Produktzyklus und über mehrere Zyklen hinweg ist mit dem Hydrosart-Typ#3-Modul ein stabiler Betriebspunkt auch bei einer Biomassebelastung von bis zu 22 kg Zellfeuchtgewicht-Äquivalent/$m^2$ (1466 g Zellfeuchtgewicht-Äquivalent/L) einzuhalten.

Im direkten Vergleich zeigt das Standard-Hydrosart-Modul deutlich geringere Leistungsdaten bei bei signifikant geringerer Biomassebelastung.

#### 4.3.2 Effekte verschiedener Rohhomogenat-Partien

Das Hydrosart-Typ#3-Modul wurde in einer unmittelbar anschließenden Versuchsserie mit einer anderen Rohhomogenat-Partie (#3) bei einer Biomassebelastung von 15 kg Zellfeuchtgewicht-Äquivalent/$m^2$ gefahren, um Effekte, die durch eine andere Rohhomogenat-Charge erzeugt werden, ebenfalls zu erfassen.

Der Retentatfluss bei Produktfahrt lag bei der neuen Rohhomogenat-Partie #3 nur noch bei konstant 35 L/h über 3 Zyklen. Der Permeatflux lag konstant zwischen 10-15 L/h $m^2$ über 3 Zyklen. Die Entfernung lag zwischen 40% und 50% und die mittlere Transmission zwischen 20% und 30%.

Die Wasserwerte nach Durchführung des Standard-Reinigungsprotokolls lagen konstant bei 1300 L/h $m^2$ Permeatwasserwert und bei 100 L/h Retentatwasserwert.

*Schlussfolgerung*

Auch bei einem Wechsel zu einer anderen Rohhomogenat-Charge (#3) wurde ein vergleichbarer Permeatflux erzielt, wie in den vorangegangenen Experimenten mit der ersten Rohhomogenat-Charge (#1). Der Retentatflux lag deutlich niedriger, als in den Experimenten zuvor. Die Reinigbarkeit des Hydrosart-Typ#3-Moduls blieb auf einem konstant hohen Niveau erhalten.

#### 4.3.3 Betriebspunktbestimmung des Hydrosart-Typ#3-Moduls

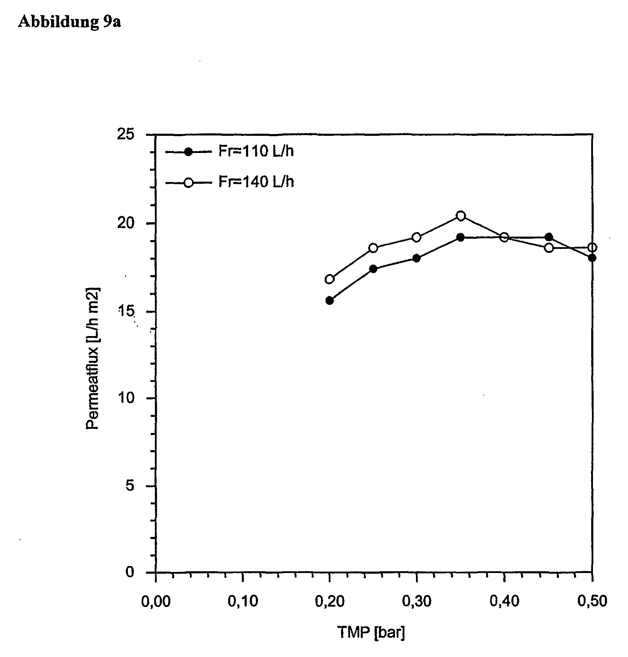

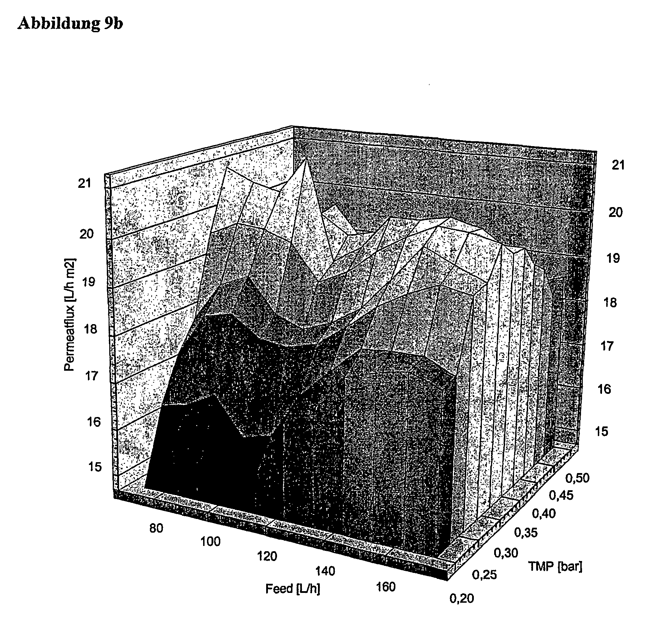

In **Abbildung 9a/9b** ist eine Betriebspunktbestimmung des Hydrosart-Typ#3-Moduls bei einer Biomassebelastung von 3 kg Zellfeuchtgewicht-Äquivalent/$m^2$ (entsprechend 230 g/L) veranschaulicht. Die Auftragung in **Abbildung 9a** entspricht der von Forman et al. gewählten, in der eine Betriebspunktbestimmung für eine Durapore-Membran (0.45 μm) bei einer Biomassekonzentration von 25 g Zellfeuchtgewicht-Äquivalent/L bis 100 g Zellfeuchtgewicht-Äquivalent/L beschrieben wurde. Vergleicht man nun diese Literaturdaten mit den in **Abbildung 9a** dargestellten Werten für das modifizierte Hydrosart-Typ#3-Modul, so fallen folgende Aspekte auf:

Der optimale TMP für das modifizierte Hydrosart-Typ#3-Modul liegt bei 0.35 bar. Eine Steigerung über dieses Optimum hinaus führt zu einer Abnahme des Permeatfluxes, was durch die Verdickung der Deckschicht auf der Membran zu erklären ist.

Der mit dem Hydrosart-Typ#3-Modul erzielte Permeatflux in diesem Experiment liegt bei etwa 20 L/h m$^2$. Die mit dem Durapore-Modul erzielten maximalen Permeatfluxe liegen bei einer Biomassekonzentration von 100 g Zellfeuchtgewicht-Äquivalent bei <8 L/h m$^2$. Der optimale TMP bei der Durapore-Membran liegt bei etwa 0.1 bar.

In **Abbildung 9b** ist die gleiche Betriebspunktbestimmung in einer dreidimensionalen Darstellung veranschaulicht. Bei einem TMP von 0.35 bar und einem Feed von 100 L/h liegt ein globales Optimum vor. Zusätzlich befinden sich lokale Optima bei einem Feed von 70 und 150 L/h bei einem TMP von ebenfalls 0.35 bar. Die optimalen Betriebspunkte sind in **Tabelle 2** noch einmal in einer Übersicht zusammengefasst.

Tabelle 2

| Globale und lokale Betriebspunkt-Optima für das Hydrosart- Typ#3-Modul. In **fetter Schrift** ist das **globale Optimum** gekennzeichnet. Die anderen beiden Betriebspunkte stellen lokale Optima dar, bei denen ebenfalls zufriedenstellende Permeatfluxe erzielt werden können. Biomassebelastung: 3 kg Zellfeuchtgewicht-Äquivalent/m$^2$. Biomassekonzentration: 230 g Zellfeuchtgewicht-Äquivalent/L Retentat. | | | | |
|---|---|---|---|---|
| TMP [bar] | Feed [L/h] | P$_{in}$ [bar] | P$_{out}$ [bar] | P$_{Perm}$ [bar] |
| 0.35 | 70 | 0.6 | 0.3 | 0.1 |
| **0.35** | **100** | **1.0** | **0.3** | **0.3** |
| 0.35 | 150 | 1.4 | 0.3 | 0.5 |

Ein Transmembrandruck von 0.35 bar stellte sich bereits früher auch für die Standard-Hydrosart-Module als optimal heraus (Daten nicht gezeigt).

### 4.3.4 Endotoxinabreicherung durch Querstrom-Mikrofiltration

In unabhängigen Versuchen wurden bei Biomassebelastungen von 15 bzw. 45 kg Zellfeuchtgewicht-Äquivalent/m$^2$ Filterfläche unter Verwendung des Hydrosart-Typ#3-Moduls (0.1 m$^2$ Filterfläche) die folgenden Ergebnisse und Endotoxinabreicherungen erzielt:

| Biomassebelastung kg/m$^2$ | Probe | Volumen [mL] | Endotoxine [EE/mL] | Gesamt-Endotoxine [EE]$_{total}$ x 10$^6$ | Abreicherungsfaktor |
|---|---|---|---|---|---|
| 15 | Retentat vor Filtration | 1300 | 240.000 | 312 | 1 |
| | Retentat nach Filtration | 1300 | 120.000 | 156 | 0.5 |
| | Permeat$_{total}$ | 5000 | 24.000 | 120 | |
| 45 | Retentat vor Filtration | 3900 | 240.000 | 936 | 1 |
| | Retentat nach Filtration | 3900 | 120.000 | 468 | 0.5 |
| | Permeat$_{total}$ | 15000 | 24.000 | 360 | |

Die Wiederfindung aller Endotoxine liegt bei ca. 90%. Die Abreicherung von Endotoxinen aus dem Retentat ins Permeat liegt bei etwa 50%, entsprechend 0.3 log-Stufen.

## 4.3.5 Maßstabsvergrößerung auf 0.6 m$^2$-Modulformat

In **Tabelle 3** sind die Ergebnisse von Versuchen zur Maßstabsvergrößerung gezeigt. Für diese Experimente wurden Hydrosart-Module vom Typ#3 mit 0.6 m$^2$ Filterfläche pro Modul eingesetzt (Sartocon-2/3-Format). Die verwendete Anlage war prinzipiell gleich aufgebaut wie in Beispiel 2 beschrieben. Der Slice-Kassettenhalter wurde allerdings gegen einen Sartocon-3 Kassettenhalter ausgetauscht und die Pumpe wurde gegen eine entsprechend größere (4 m$^3$/h) Kreiskolbenpumpe mit doppeltwirksamer Gleitringdichtung (Fa. Johnson) ausgetauscht. Das Retentatvorlage-Volumen wurde auf 5 L erhöht und das Totvolumen der Anlage lag bei 2.5 L. Entsprechend wurden die anderen Volumina (z.B. Reinigungslösungen und Waschpuffervolumina) angepasst.

Tabelle 3

| Zusammenfassung der Ergebnisse von Experimenten zur Maßstabsvergrößerung der Mikrofiltration von *E. coli* Rohhomogenat. Beispielhaft wurden Interleukin-4 R121D Y124D Protein- einschlusskörper verwendet. | | |
|---|---|---|
| Die Daten repräsentieren Mittelwerte aus jeweils 3 unabhängigen Experimenten, die unter gleichen, standardisierten Bedingungen durchgeführt wurden (Filtrations- und Reinigungsbedingungen s. Beispiel 2). | | |
| **Kriterium** | **Hydrosart-Typ#3-Modul (0.1 m$^2$, Slice)** | **Hydrosart-Typ#3-Modul (0.6 m$^2$, Sartocon-3)** |
| Skalierungsfaktor | 1 | 6 |
| Mittlerer Retentatfluss [L/h] bei Produktfahrt | 35 ±5 | 210 ±20 |
| Mittlerer Permeatflux [L/h m$^2$] bei Produktfahrt | 15 ±5 | 20 ±10 |
| Entfernung [%] | 60 ±20 | 50 ±5 |
| Transmission [%] | 30 ±5 | 40 ±5 |
| Retentatwasserwert [L/h] | 100 ±10 | 600 ±50 |
| Permeatwasserwert [L/h] | 1300 ±200 | 1300 ±200 |

*Schlussfolgerung*

Die in **Tabelle 3** zusammengestellten Daten verdeutlichen, dass das Hydrosart-Typ#3-Modul linear skalierbar ist, wobei die Leistungsdaten des Sartocon-3-Modulformats sich entsprechend zu denen des Slice-Modulformats bewegen. Vergrößerung um den Skalierungsfaktor 6 hat zur Folge, dass der Retentatfluss bei Produktfahrt um exakt den Faktor 6 angehoben wird. Entsprechend wird der Retentatwasserwert um den Faktor 6 erhöht. Die anderen Leistungsdaten, wie Permeatflux bei Produktfahrt, Permeatwasserwert, Entfernung und Transmission bleiben in den Grenzen der jeweiligen Bestimmungsgenauigkeit gleich.

**Zitierte Literaturstellen**

Apeler H, Wehlmann H (1998): Plasmids, their construction and their use in the manufacture of Interleukin-4 and Interleukin-4 muteins. European Patent application EP 00100129.6, filed January 7, 2000.
Bailey SM & Meagher MM (1997): J. Membr. Sci. 131: 29-38
Bailey SM and Meagher MM (1997): Biotechnol. Bioeng. 56(3): 304-310
Blum P et al. (1992): Bio/Technology 10: 301-304
Bowden GA, Paredes AM, Georgiou G (1991): Bio/Technology 9: 725-730
Forman SM, DeBernardez ER, Feldberg RS, Swartz RW (1990): J. Membr. Sci. 48: 263-279
Marston FAO (1986): Biochem. J. 240: 1-12
Meagher MM, Barlett RT, Rai VR, Khan FR (1994): Biotechnol. Bioeng. 43: 969-977
Meyeroltmanns F (1991): BioTec 5: 918-921
Riesenberg D, Menzel K, Schulz V, Schumann K, Veith G, Zuber G, Knorre WA (1990): Appl. Microbiol. Biotechnol. 34: 77-82
Schoemaker JM, Brasnett AH, Marston FAO (1985): EMBO J 4: 775-780

Schoner RG, Ellis FL, Schoner BE (1985): Biotechnology 3: 151-154

Sebald W (1998): Therapeutic agents which are antagonists or partial agonists of human Interleukin-4, US Patent 5,723,118, 3 May 1998. Human IL-4 mutant proteins, Eur. Patent 0 613 499 B1, 2 November 1998.

Sharma SK (1986): J. Biotechnol. 4: 119-124

Taylor G, (1986): Bio/Technology 4: 553-557

**Abbildungstexte**

**Abbildung 1**  Diafiltration von *E. coli* Rohhomogenat (Partie #1) mit Proteineinschlusskörpern von Interleukin-4 R121D Y124D. 1. Produktzyklus mit einem fabrikneuen **Hydrosart-Slice Standard-Modul** (0.1 m$^2$) 3051860601 W--SG, Lot nr. 96108741/13. 300 g Zellfeuchtgewicht-Äquivalent (3 kg Zellfeuchtgewicht-Äquivalent/m$^2$). 2.55 L Gesamt-Retentatvolumen (117 g Zellfeuchtgewicht-Äquivalent/L).

**Abbildung 2**  Diafiltration von *E. coli* Rohhomogenat (Partie #1) mit Proteineinschlusskörpern von Interleukin-4 R121D Y124D. 1. Produktzyklus mit einem fabrikneuen, **modifizierten Hydrosart-Slice Modul Typ#1** (0.1 m$^2$), Lot nr. 98025101. 300 g Zellfeuchtgewicht-Äquivalent (3 kg Zellfeuchtgewicht-Äquivalent/m$^2$). 2.55 L Gesamt-Retentatvolumen (117 g Zellfeuchtgewicht-Äquivalent/L).

**Abbildung 3a**  **Retentatfluss (L/h) und mittlerer Permeatflux (L/h m$^2$) bei Produktfahrt** in Abhängigkeit von der Zykluszahl. Diafiltration von *E. coli* Rohhomogenat (Partie #1) mit Proteineinschlusskörpern von Interleukin-4 R121D Y124D. 1. bis 9. Produktzyklus mit einem fabrikneuen, **modifizierten Hydrosart-Slice Modul Typ#1** (0.1 m$^2$), Lot nr. 98025101. 300 g Zellfeuchtgewicht-Äquivalent (3 kg Zellfeuchtgewicht-Äquivalent/m$^2$). 2.55 L Gesamt-Retentatvolumen (117 g Zellfeuchtgewicht-Äquivalent/L).

**Abbildung 3b**  **Wasserwerte vor und nach Produktfahrt.** Retentatfluss (L/h) und Permeatflux (L/h m$^2$) in Abhängigkeit von der Zykluszahl. Diafiltration von *E. coli* Rohhomogenat (Partie #1) mit Proteineinschlusskörpern von Interleukin-4 R121D Y124D. 1. bis 9. Produktzyklus mit einem fabrikneuen, **modifizierten Hydrosart-Slice Modul Typ#1** (0.1 m$^2$), Lot nr. 98025101. 300 g Zellfeuchtgewicht-Äquivalent (3 kg Zellfeuchtgewicht-Äquivalent/m$^2$). 2.55 L Gesamt-Retentatvolumen (117 g Zellfeuchtgewicht-Äquivalent/L).

**Abbildung 4**  Diafiltration von *E. coli* Rohhomogenat (Partie #1) mit Proteineinschlusskörpern von Interleukin-4 R121D Y124D. 1. Produktzyklus mit einem fabrikneuen, **modifizierten Hydrosart-Slice Modul Typ#2** (0.05 m$^2$), Lot nr. 98055101. 300 g Zellfeuchtgewicht-Äquivalent (3 kg Zellfeuchtgewicht-Äquivalent/m$^2$). 1.3 L Gesamt-Retentatvolumen (230 g Zellfeuchtgewicht-Äquivalent/L).

**Abbildung 5a**  **Retentatfluss (L/h) und mittlerer Permeatflux (L/h m$^2$) bei Produktfahrt** in Abhängigkeit von der Zykluszahl. Diafiltration von *E. coli* Rohhomogenat (Partie #1) mit Proteineinschlusskörpern von Interleukin-4 R121D Y124D. 1. bis 7. Produktzyklus mit einem fabrikneuen, **modifizierten Hydrosart-Slice Modul Typ#2** (0.05 m$^2$), Lot nr. 98055101. 300 g Zellfeuchtgewicht-Äquivalent (3 kg Zellfeuchtgewicht-Äquivalent/m$^2$). 1.3 L Gesamt-Retentatvolumen (230 g Zellfeuchtgewicht-Äquivalent/L).

**Abbildung 5b**  **Wasserwerte vor und nach Produktfahrt.** Retentatfluss (L/h) und Permeatflux (L/h m$^2$) in Abhängigkeit von der Zykluszahl. Diafiltration von *E. coli* Rohhomogenat (Partie #1) mit Proteineinschlusskörpem von Interleukin-4 R121D Y124D. 1. bis 7. Produktzyklus mit einem fabrikneuen, **modifizierten Hydrosart-Slice Modul Typ#2** (0.05 m$^2$), Lot nr. 98055101. 300 g Zellfeuchtgewicht-Äquivalent (3 kg Zellfeuchtgewicht-Äquivalent/m$^2$). 1.3 L Retentatvolumen (230 g Zellfeuchtgewicht-Äquivalent/L).

**Abbildung 6**  Diafiltration von *E. coli* Rohhomogenat (Partie #2) mit Proteineinschlusskörpem von Interleukin-4 R121D Y124D. 1. Produktzyklus mit einem fabrikneuen, **modifizierten Hydrosart-Slice Modul Typ#3** (0.1 m$^2$), Lot nr. 99015111. 300 g Zellfeuchtgewicht-Äquivalent (3 kg Zellfeuchtgewicht-Äquivalent/m$^2$). 1.3 L Retentatvolumen (230 g Zellfeuchtgewicht-Äquivalent/L).

**Abbildung 7a**  **Retentatfluss (L/h) und mittlerer Permeatflux (L/h m$^2$) bei Produktfahrt** in Abhängigkeit von der Zykluszahl bei Steigerung der Biomassebelastung der Membran. Diafiltration von *E. coli* Rohhomogenat (Partie #2) mit Proteineinschlusskörpern von Interleukin-4 R121D Y124D. 1. bis 5. Produktzyklus mit einem fabrikneuen, **modifizierten Hydrosart-Slice Modul Typ#3** (0.1 m$^2$), Lot nr. 99015111. 3 kg Zellfeuchtgewicht-Äquivalent/m$^2$, 5 kg/m$^2$, 10 kg/m$^2$, 15 kg/m$^2$ entsprechend 230 g Zellfeuchtgewicht-Äquivalent/L, 384 g/L, 770 g/L, 1153 g/L.

**Abbildung 7b**     **Wasserwerte vor und nach Produktfahrt.** Retentatfluss (L/h) und Permeatflux (L/h m$^2$) in Abhängigkeit von der Zykluszahl. Diafiltration von *E. coli* Rohhomogenat (Partie #2) mit Proteineinschlusskörpern von Interleukin-4 R121D Y124D. 1. bis 5. Produktzyklus mit einem fabrikneuen, **modifizierten Hydrosart-Slice Modul Typ#3** (0.1 m$^2$), Lot nr. 99015111. 3 kg Zellfeuchtgewicht-Äquivalent/m$^2$, 5 kg/m$^2$, 10 kg/m$^2$, 15 kg/m$^2$ entsprechend 230 g Zellfeuchtgewicht-Äquivalent/L, 384 g/L, 770 g/L, 1153 g/L.

**Abbildung 8**     **Stufenweise Erhöhung der Biomassebelastung.**

**A.** Diafiltration von *E. coli* Rohhomogenat (Partie #2) mit Proteineinschlusskörpern von Interleukin-4 R121D Y124D mit einem **modifizierten Hydrosart-Slice Modul Typ#3** (0.1 m$^2$), Lot nr. 99015111. Startbedingungen: 1500 g Zellfeuchtgewicht-Äquivalent (15 kg Zellfeuchtgewicht-Äquivalent/m$^2$). 1.3 L Retentatvolumen (1153 g Zellfeuchtgewicht-Äquivalent/L). Schrittweise Erhöhung der Biomassebelastung der Membran zu den in der Grafik indizierten Zeitpunkten während der laufenden Diafiltration auf 20, 22, 24 und schließlich 26 kg Zellfeuchtgewicht-Äquivalent/m$^2$. Durch die Volumenänderung im Retentat ergeben sich daraus entsprechend 1176 g Zellfeuchtgewicht-Äquivalent/L, 1466 g/L, 1600 g/L und schließlich 1733 g/L.

**B.** Diafiltration von *E. coli* Rohhomogenat (Partie #3) mit Proteineinschlusskörpern von Interleukin-4 R121D Y124D mit einem **Standard-Hydrosart-Slice** Modul (0.1 m$^2$), Lot nr. 96108741/008. Startbedingungen: 300 g Zellfeuchtgewicht-Äquivalent (3 kg Zellfeuchtgewicht-Äquivalent/m$^2$). 1.3 L Retentatvolumen (230 g Zellfeuchtgewicht-Äquivalent/L). Schrittweise Erhöhung der Biomassebelastung der Membran zu den in der Grafik indizierten Zeitpunkten während der laufenden Diafiltration auf 5 kg und schließlich 10 kg Zellfeuchtgewicht-Äquivalent/m$^2$. Danach wurde aufgrund der geringen Permeatfluxe das Experiment abgebrochen. Das Retentatvolumen wurde auf konstant 1.3 L gehalten. Daraus ergeben sich entsprechend Biomassekonzentration im Retentat zu 384 g Zellfeuchtgewicht-Äquivalent/L und 769 g/L.

**Abbildung 9a**     **Betriebspunktbestimmung des modifizierten Hydrosart-Typ#3-Moduls.** Biomassebelastung 3 kg Zellfeuchtgewicht-Äquivalent/m$^2$ (230 g Zellfeuchtgewicht-Äquivalent/L), Rohhomogenat Partie #2. Der Permeatflux ist in Abhängigkeit vom Transmembrandruck (TMP) aufgetragen. Der Retentatfluss ($F_r$) wurde bei ca. 110 L/h bzw. ca. 140 L/h fixiert. Das Experiment wurde unter vollständiger Rezyklierung aller Volumenströme durchgeführt.

**Abbildung 9b**     **Betriebspunktbestimmung des modifizierten Hydrosart-Typ#3-Moduls.** Biomassebelastung: 3 kg Zellfeuchtgewicht-Äquivalent/m$^2$ (entsprechend 230 g Zellfeuchtgewicht-Äquivalent/L), Rohhomogenat Partie #2. Der Permeatflux ist in Abhängigkeit vom Transmembrandruck (TMP) und Feed (=Retentatfluss+Permeatfluss) aufgetragen. Das Experiment wurde unter vollständiger Rezyklierung aller Volumenströme durchgeführt.

**Abbildung 10**     Schematische Darstellung des Versuchsaufbaus einer Querstrom-Mikrofiltrationsanlage

| # | Bedeutung |
|---|---|
| 1 | Retentatvorlage (gerührt) mit Füllstandssensor (Steuerung für Waschpuffer-Pumpe (9)) |
| 2 | Kreiskolbenpumpe |
| 3 | Kassettenhalterung (Sartocon-Slice-Format, Sartorius AG, Deutschland) ausgerüstet mit Hydrosart-Slice-Kassette (0.1 m$^2$ Filtrationsfläche) |
| 4 | Drosselventil Retentatausgang |
| 5 | Drosselventil Permeatausgang |

(fortgesetzt)

| # | Bedeutung |
|---|-----------|
| 6 | Permeatnachlage |
| 7 | Manometer R(in)=7a; R(out)=7b; P=7c |
| 8 | Waschpuffer-Pumpe |
| 9 | Waschpuffer-Vorlage |

Das Totvolumen der verwendeten Anlage beträgt 0.55 L.

**Patentansprüche**

1. Verfahren zur Reinigung und/oder zur Konzentrierung von Proteineinschlusskörpem in einer Lösung, welches **dadurch gekennzeichnet ist, dass** die Proteineinschlusskörper enthaltende Lösung tangential an einer oder mehreren semipermeablen Membranen vorbeigeleitet wird, so dass die Proteineinschlusskörper von den Membranen zurückgehalten werden und Substanzen oder Bestandteile mit einem kleineren Molekulargewicht bzw. Partikeldurchmesser durchtreten können, wobei eine gereinigte und/oder konzentrierte Proteineinschlusskörper-Lösung erhalten wird, wobei auf der semipermeablen Membran eine offenmaschige Matrix liegt, die aus sich kreuzenden Längs- und Querfäden aufgebaut ist derart, dass die benachbarten Längs- und Querfäden jeweils zueinander einen 5- bis 15fachen Abstand ihrer Dicke einnehmen, welche im Bereich von 150 bis 600 µm liegt.

2. Verfahren nach Anspruch 1, **dadurch gekennzeichnet, dass** die Biomassebelastung der Membran größer als 65 g Zellfeuchtgewicht-Äquivalent/m$^2$ ist.

3. Verfahren nach Anspruch 1, **dadurch gekennzeichnet, dass** die Biomassebelastung der Membran größer als 650 g Zellfeuchtgewicht-Äquivalent/m$^2$ ist.

**Claims**

1. Process for purifying and/or concentrating inclusion bodies in a solution, which is **characterized in that** the inclusion body-containing solution is directed tangentially past one or more semipermeable membranes so that the membranes retain the inclusion bodies and that substances or components having a relatively small molecular weight or particle diameter can pass through, thus obtaining a purified and/or concentrated inclusion body solution, and on the semipermeable membrane a wide-meshed matrix is located which is constructed of intersecting longitudinal and transverse filaments such that the adjacent longitudinal and transverse filaments are, in each case, at a distance from one another which is 5 to 15 times greater than their thickness which is in the range from 150 to 600 µm.

2. Process according to Claim 1, **characterized in that** the biomass load of the membrane is greater than 65 g of wet cell mass equivalent/m$^2$.

3. Process according to Claim 1, **characterized in that** the biomass load of the membrane is greater than 650 g of wet cell mass equivalent/m$^2$.

**Revendications**

1. Procédé de purification et/ou de concentration de corps d'inclusion protéiniques dans une solution, procédé **caractérisé en ce qu'**on fait passer la solution contenant des corps d'inclusion protéiniques tangentiellement sur une ou plusieurs membranes semi-perméables, de manière que les corps d'inclusion protéiniques soient retenus par les membranes et que des substances ou constituants de plus faible poids moléculaire ou de plus faible diamètre des particules puissent passer à travers les membranes, et on obtient ainsi une solution purifiée et/ou concentrée de corps d'inclusion protéiniques, la membrane semi-perméable portant une matrice à mailles ouvertes qui est constituée de fils longitudinaux et transversaux entrecroisés de telle sorte que les fils longitudinaux et transversaux ou voisins se trouvent les uns par rapport aux autres à une distance de 5 à 15 fois leur épaisseur, laquelle se situe dans la plage de 150 à 600 μm.

2.  Procédé suivant la revendication 1, **caractérisé en ce que** la charge de biomasse de la membrane est supérieure à 65 g d'équivalent en poids humide de cellules par m$^2$.

3.  Procédé suivant la revendication 1, **caractérisé en ce que** la charge de biomasse de la membrane est supérieure à 650 g d'équivalent en poids humide de cellules par m$^2$.

# Abbildungen

## Abbildung 1

**Abbildung 2**

**Abbildung 3a**

**Abbildung 3b**

**Abbildung 4**

**Abbildung 5a**

**Abbildung 5b**

**Abbildung 6**

**Abbildung 7a**

| massebelastung: | 3 | 5 | 10 | 15 | 15 |

Zellfeuchtgewicht-Äquivalent/m$^2$)

**Abbildung 7b**

**Biomassebelastung:**
(kg Zellfeuchtgewicht-Äquivalent/m$^2$)

| 3 | 5 | 10 | 15 | 15 |

**Biomassebelastung:**
(kg Zellfeuchtgewicht-Äquivalent/m$^2$)

| 3 | 5 | 10 | 15 | 15 |

**Abbildung 8**

A

B

**Abbildung 9a**

**Abbildung 9b**

**Abbildung 10**